# EUROPEAN PATENT APPLICATION

(11) **EP 1 600 166 A1**
(43) Date of publication of application: **30.11.2005**
(21) Application number: 04702450.0
(22) Date of filing: 15.01.2004
(51) Int. Cl.: A61K 45/00, A61K 31/553, A61P 19/00, A61P 21/00, A61P 43/00

(54) **SKELETAL MUSCLE PROTECTING AGENT**

(30) Priority: 17.01.2003 JP 2003010125; 31.03.2003 JP 2003093591
(71) Applicant: Takeda Pharmaceutical Company Limited, Osaka-shi, Osaka 541-0045 (JP)
(72) Inventor: TOZAWA, Ryuichi, Toyonaka-shi, Osaka 560-0021 (JP); NISHIMOTO, Tomoyuki, Suita-shi, Osaka 565-0862 (JP)
(74) Representative: von Kreisler, Alek
(86) International application number: PCT/JP2004/000234
(87) International publication number: WO 2004/064865

(57) **Abstract**

The present invention provides a novel drug which is useful as a skeletal muscle protecting agent comprising a compound having inhibitory activity against squalene synthase or a salt thereof, or a prodrug thereof.

## Description

### Technical Field

The present invention relates to a skeletal muscle protecting agent comprising a compound having inhibitory activity against squalene synthase or a salt thereof, or a prodrug thereof.

### Background Art

The skeletal muscle is an important muscle which is attached to the bone and involved in exercise of the body, but necrosis or myolysis of the skeletal muscle occurs by various factors, for example, ischemia, labor, excessive exercise, trauma (bruise, skeletal muscle bleeding, electric shock), burn, malignant hyperthermia, malignant syndrome, metabolic myopathy, inflammatory myopathy, muscular dystrophy, infection, poisoning, abnormal metabolism, hyperthermia and the like. Further, myalgia may occur by toxicity of drugs, for example, HMG-CoA reductase inhibitors, cyclosporin, fibrates and the like, and if it becomes serious, rhabdomyolysis is developed. In such case, administration of the medicine which is considered to be causal is stopped, and maintaining the rest, enough fluid replacement is carried out. There is no effective treatment for muscular lesion, and treatment for the complication or fundamental disease is the focus.

On the other hand, it has been known that a compound having inhibitory activity against squalene synthase is useful as a preventive and/or therapeutic agent for hyperlipidemia or atherosclerosis and the like, a triglyceride-lowering agent, a lipid-lowering agent, a high density lipoprotein cholesterol-elevating agent, an antifungal agent and the like (JP-A Nos. 6-239843, 8-157369, 9-136880, 2002-080468, and 2002-205956). For the action for the skeletal muscle, there has been only a report that the cytotoxicity is less likely to be manifested than that of the HMG-CoA reductase inhibitor (Oliver P. Flint et al), pp. 91-98, Vol. 145, "Toxicology and Applied Pharmacology", 1997), but no report that they show protecting action for the skeletal muscle in vitro or in vivo. Further, it has been known that a compound having inhibitory activity against squalene synthase increases ubiquinone (WO03/002147), but it has been shown that muscular toxicity of HMG-CoA reductase inhibitor has nothing to do with ubiquinone (William H. Schaefer et al, doi:10.1016/j.taap. August 13, 2003 "Toxicology and Applied Pharmacology").

### Objects of the Invention

A medicine which can protect the skeletal muscle from various factors, particularly, from cytotoxicity of an HMG-CoA reductase inhibitor has not been known so far. Then, at present, it is desired to develop a novel medicine which is clinically useful.

### Disclosure of the Invention

Under the above circumstances, the present inventors have studied intensively, and as a result, found unexpectedly for the first time that a compound having squalene synthase inhibitory activity is clinically useful as a medication for protecting the skeletal muscle, which resulted in the completion of the present invention.

That is, the present invention relates to:
(1) A skeletal muscle protecting agent comprising a compound having inhibitory activity against squalene synthase or a salt thereof, or a prodrug thereof;
(2) The agent as described in the above-mentioned (1) which is a skeletal muscle protecting agent which protects skeletal muscle from cell disorder;
(3) The agent as described in the above-mentioned (1) which is a skeletal muscle protecting agent which protects skeletal muscle from cytotoxicity of other medicines;
(4) The agent as described in the above-mentioned (3), wherein the other medicine is an HMG-CoA reductase inhibitor;
(5) The agent as described in the above-mentioned (1) which is a preventive and/or therapeutic agent for myalgia or rhabdomyolysis;
(6) The agent as described in the above-mentioned (1), wherein the compound having inhibitory activity against squalene synthase is a compound represented by the formula: 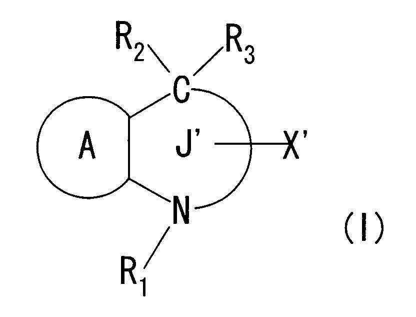 wherein R₁ is a hydrogen atom or an optionally substituted hydrocarbon group, R₂ and R₃ are the same or different and a hydrogen atom, an optionally substituted hydrocarbon group or an optionally substituted heterocyclic group, X' is a substituent comprising an optionally esterified carboxyl group, an optionally substituted carbamoyl group, an optionally substituted hydroxy group, an optionally substituted amino group or an optionally substituted heterocyclic residue having a hydrogen atom which can be deprotonated, Ring A is an optionally substituted benzene ring or an optionally substituted heterocyclic ring, Ring J' is a 7- or 8-membered heterocyclic ring having 3 or less hetero atoms, as atoms constituting a ring, and Ring J' may further have a substituent in addition to R₁, R₂, R₃ and X' ;
(7) The agent as described in the above-mentioned (1), wherein the compound having inhibitory activity against squalene synthase is a compound represented by the formula: 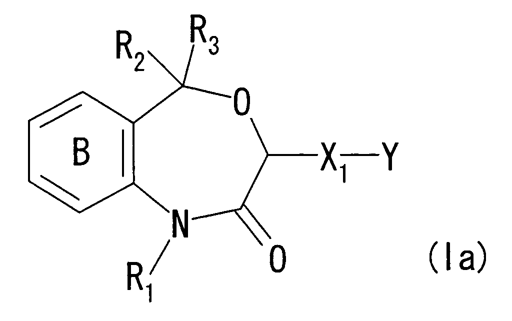 wherein R₁ is a hydrogen atom or an optionally substituted hydrocarbon group, R₂ and R₃ are the same or different and a hydrogen atom, an optionally substituted hydrocarbon group or an optionally substituted heterocyclic group, X₁ is a bond or divalent atomic chain, Y is an optionally esterified carboxyl group, an optionally substituted carbamoyl group, an optionally substituted hydroxy group, an optionally substituted amino group or an optionally substituted heterocyclic residue having a hydrogen atom which can be deprotonated, and Ring B is an optionally substituted benzene ring;
(8) The agent as described in the above-mentioned (1), wherein the compound having inhibitory activity against squalene synthase is a compound represented by the formula: 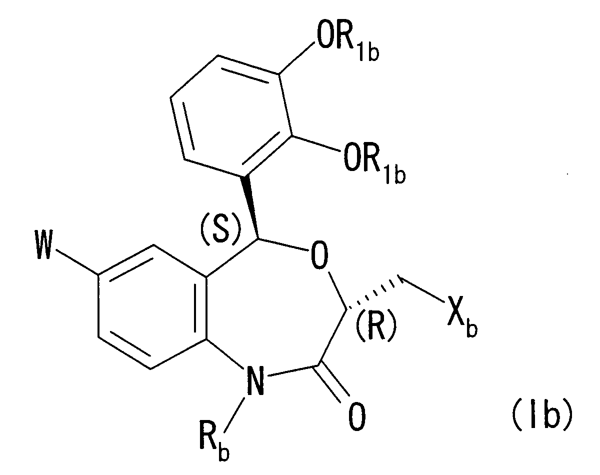 wherein R_{b} is a lower alkyl group optionally substituted with an optionally substituted hydroxy group, X_{b} is an optionally substituted carbamoyl group or an optionally substituted heterocyclic group having a hydrogen atom which can be deprotonated, R_{1b} is a lower alkyl group and W is a halogen atom;
(9) The agent as described in the above-mentioned (8), wherein R_{b} is C₁₋₆ alkyl which may have 1 to 3 substituents selected from a hydroxy group, acetyloxy, propionyloxy, t-butoxycarbonyloxy, palmitoyloxy, dimethylaminoacetyloxy and 2-aminopropionyloxy;
(10) The agent as described in the above-mentioned (8), wherein R_{1b} is methyl;
(11) The agent as described in the above-mentioned (8), wherein W is a chlorine atom;
(12) The agent as described in the above-mentioned (8), wherein X_{b} is a group represented by the formula: 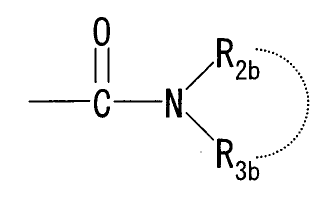 wherein R_{2b} and R_{3b} are each a hydrogen atom, an optionally substituted hydrocarbon group, an optionally substituted heterocyclic group or an acyl group, or R_{2b} and R_{3b} may form, together with the adjacent nitrogen atom, an optionally substituted 5- or 6-membered nitrogen-containing heterocyclic ring which may contain 1 to 3 hetero atoms selected from a nitrogen atom, a sulfur atom and an oxygen atom, as atoms constituting a ring;
(13) The agent as described in the above-mentioned (8), wherein X_{b} is a group represented by the formula: 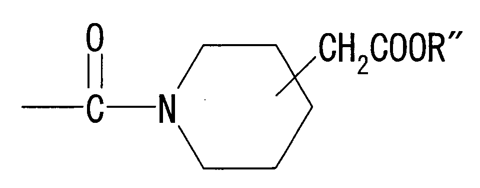 wherein R" is a hydrogen atom or C₁₋₄ alkyl;
(14) The agent as described in the above-mentioned (1), wherein the compound having inhibitory activity against squalene synthase is a compound represented by the formula: 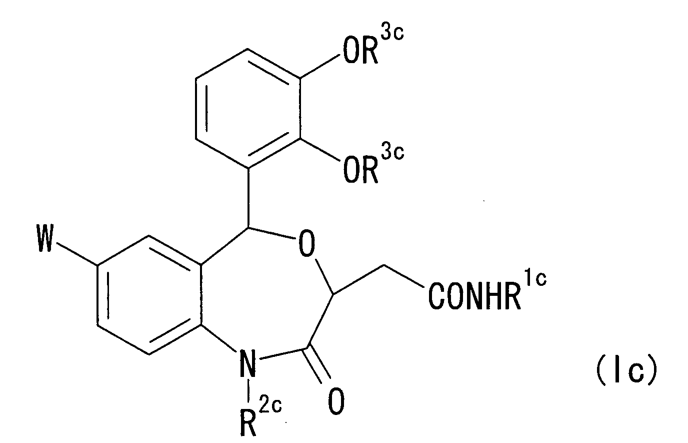 wherein R^{1c} is an optionally substituted 1-carboxyethyl group, an optionally substituted carboxy-C₃₋₆ straight-chain alkyl group, an optionally substituted C₃₋₆ straight-chain alkyl-sulfonyl group, an optionally substituted (carboxy-C₅₋₇ cycloalkyl)-C₁₋₃ alkyl group, or a group represented by the formula -X^{1c}-X^{2c}-Ar-X^{3c}-X^{4c}-COOH (wherein X^{1c} and X^{4c} are each a bond or an optionally substituted C₁₋₄ alkylene group, X^{2c} and X^{3c} are each a bond, -0- or -S-, and Ar is an optionally substituted divalent aromatic cyclic group, provided that X^{2c} is a bond when X^{1c} is a bond and X^{3c} is a bond when X^{4c} is a bond), R^{2c} is a C₃₋₆ alkyl group optionally substituted with an alkanoyloxy group and/or a hydroxy group, R^{3c} is a lower alkyl group and W is a halogen atom (provided that R^{2c} is a C₃₋₆ alkyl group having an alkanoyloxy group and/or a hydroxy group when R^{1c} is a substituted 1-carboxyethyl group, a substituted carboxy-C₃₋₆ straight-chain alkyl group, a 4-carboxycyclohexylmethyl group or a 4-carboxymethylphenyl group);
(15) The agent as described in the above-mentioned (14), wherein R^{2c} is a C₃₋₆ alkyl group which may have 1 to 3 substituents selected from a hydroxy group, acetoxy, propionyloxy, t-butoxycarbonyloxy and palmitoyloxy;
(16) The agent as described in the above-mentioned (14), wherein R^{3c} is a methyl group;
(17) The agent as described in the above-mentioned (14), wherein W is a chlorine atom;
(18) The agent as described in the above-mentioned (14), wherein the 3-position has an R-configuration and the 5-position has an S-configuration;
(19) The agent as described in the above-mentioned (1) wherein the compound having inhibitory activity against squalene synthase is N-[[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetyl]piperidine-4-acetic acid or N-[[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl]piperidine-4-acetic acid;
(20) A skeletal muscle protecting agent comprising a compound having an action of suppressing the decrease of a geranylgeranylated metabolite in a muscular cell, or a salt thereof, or a prodrug thereof;
(21) A method for protecting skeletal muscle, comprising administering an effective amount of a compound having inhibitory activity against squalene synthase, or a salt thereof, or a prodrug thereof to a mammal;
(22) A method for protecting skeletal muscle, comprising administering an effective amount of a compound having an action of suppressing the decrease of a geranylgeranylated metabolite in a muscular cell, or a salt thereof, or a prodrug thereof to a mammal;
(23) Use of a compound having inhibitory activity against squalene synthase, or a salt thereof, or a prodrug thereof for manufacturing a skeletal muscle protecting agent;
(24) Use of a compound having an action of suppressing the decrease of a geranylgeranylated metabolite in a muscular cell, or a salt thereof, or a prodrug thereof for manufacturing a skeletal muscle protecting agent; and the like.

As for the "compound having inhibitory activity against squalene synthase" used in the present invention, any compound having inhibitory activity against squalene synthase may be included, for example, squalenestatins (e.g., USP Nos. 5506262, 5430055, 5409950, 5369125, JP-A Nos. 7-173166, 9-124655, 9-227566, "Annual Review of Microbiology", Vol.49, pp. 607-639, 1995, "Journal of Medicinal Chemistry", Vol.38, pp. 3502-3513, 1995, "Journal of Medicinal Chemistry", Vol.39, pp. 207-216, 1996, "Journal of Medicinal Chemistry", Vol.39, pp. 1413-1422, 1996, etc.), a phosphate compound and a carboxylic acid compound of a substrate analog (e.g., USP Nos. 5374628, 5441946, 5428028, JP-A No. 7-041554, W095/04025, "Journal of Medicinal Chemistry", Vol.38, pp.2596-2605, 1995, "Arzniemittel-Forschung Drug Research", Vol.46, pp. 759-762, 1996, "Journal of Medicinal Chemistry", Vol.31, pp. 1869-1871, 1988, "Journal of Medicinal Chemistry", Vol.39, pp. 657-660, 1996, "Journal of Medicinal Chemistry", Vol.39, pp. 661-664, 1996), carboxylic acid derivatives (e.g., WO97/40006, WO96/33159, WO95/21834, WO97/48701, EP-A Nos. 645377, 645378, 814080, 790235, JP-A Nos. 7-173120, 10-316634, 10-298134, 10-298177, 10-316617, 9-136880, WO2000/00458, WO2001/98282, WO98/29380, "Bioorganic Medicinal Chemistry Letters", Vol.5, pp. 1989-1994, 1995, "Bioorganic Medicinal Chemistry Letters", Vol.6, pp. 463-466, 1996, "Journal of Medicinal Chemistry", Vol.40, pp. 2123-2125, 1997, etc.), an amine-based compound such as quinuclidine derivatives (e.g., USP Nos. 5385912, 5494918, 5395846, 5451596, JP-A Nos. 8-134067, 2000-169474, 10-152453, 2000-502716, WO94/03541 WO 94/05660, WO95/35295, WO96/26938, WO95/31458, WO95/00146, WO97/25043, WO98/12170, etc.), and Zaragozic acids, particularly, a compound represented by the formula: 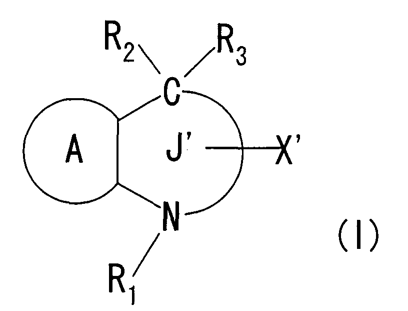 wherein, R₁ is a hydrogen atom or an optionally substituted hydrocarbon group, R₂ and R₃ are the same or different and a hydrogen atom, optionally substituted hydrocarbon group or an optionally substituted heterocyclic group, X' is a substituent comprising an optionally esterified carboxyl group, an optionally substituted carbamoyl group, an optionally substituted hydroxy group, an optionally substituted amino group or an optionally substituted heterocyclic residue having a hydrogen atom which can be deprotonated, Ring A is an optionally substituted benzene ring or an optionally substituted heterocyclic ring, Ring J' is a 7- to 8-membered heterocyclic ring containing 3 or less hetero atoms, as atoms constituting a ring, and Ring J' may further have a substituent in addition to R₁, R₂, R₃, and X'; a compound represented by the formula: 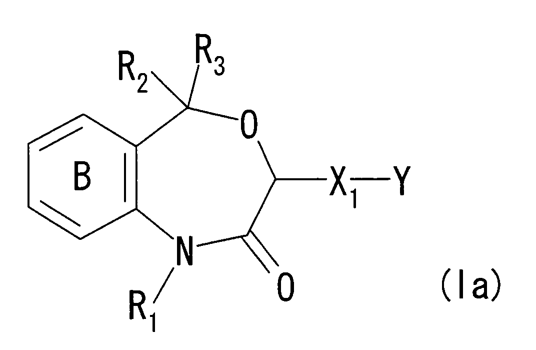 wherein, R₁ is a hydrogen atom or an optionally substituted hydrocarbon group, R₂ and R₃ are the same or different and a hydrogen atom, optionally substituted hydrocarbon group or optionally substituted heterocyclic group, X₁ is a bond or divalent atomic chain, Y is an optionally esterified carboxyl group, an optionally substituted carbamoyl group, and an optionally substituted hydroxy group, an optionally substituted amino group, an optionally substituted heterocyclic residue having a hydrogen atom which can be deprotonated, Ring B is an optionally substituted benzene ring; or the like is preferably used.

Examples of other squalene synthase inhibitors include A-104109 (Abbott Laboratories), 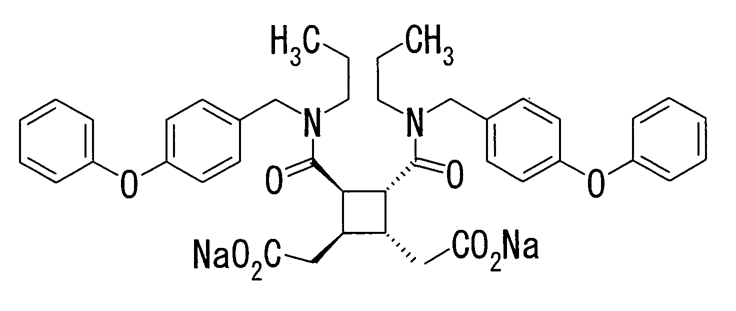 F-10863-A (Zaragozic acid D3, Sankyo Co., Ltd.), ER-28448, ER-27856 (ER-28448 prodrug) and quinuclidine derivatives (Eisai), 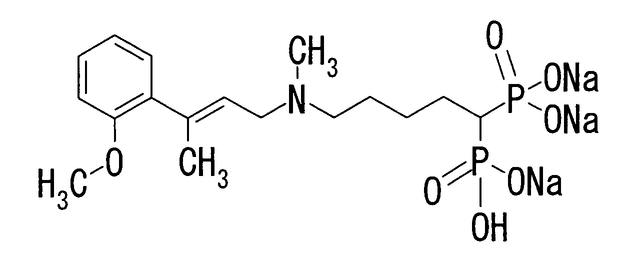 RPR-107393 and RPR-101821 (Aventis Pharma Ltd.), 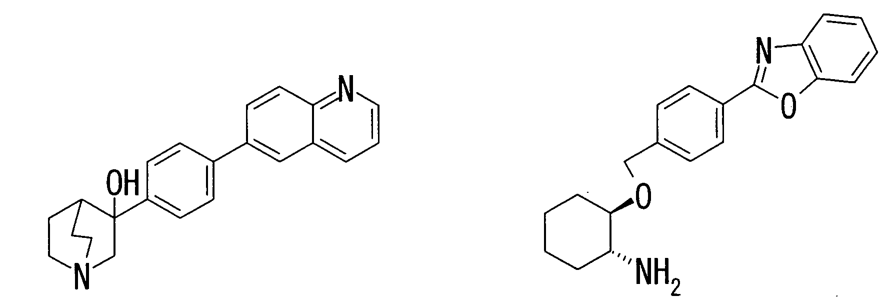 thiadiazole derivatives (NovoNordisk), 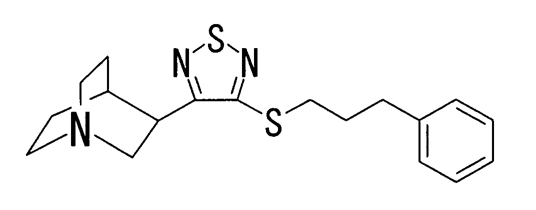 isopropylamine derivatives (Yamanouchi Pharmaceutical Co., Ltd.), 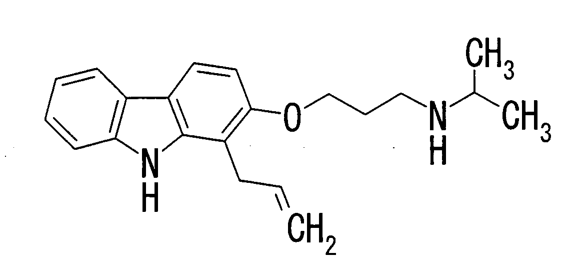 isoquinuclidine derivatives (Kotobuki pharmaceutical Co., Ltd.) 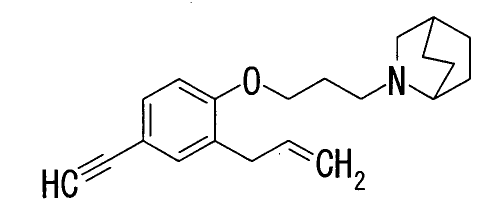 malonic acid derivatives (Nippon Kayaku Co., Ltd.), and 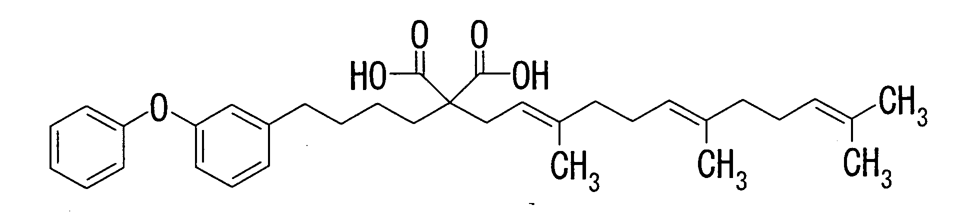 propionyl derivatives (Daiichi Pharmaceutical Co., Ltd.), 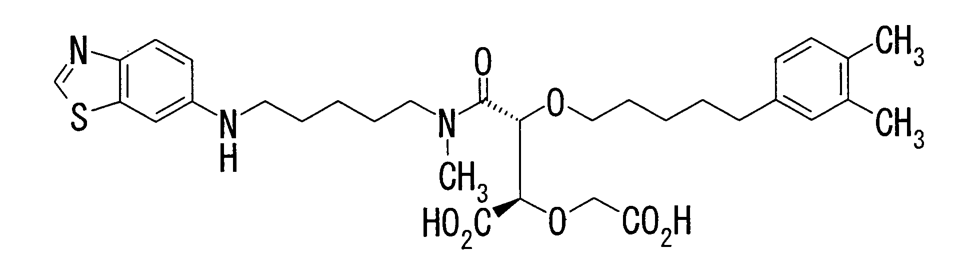 and such squalene synthase inhibitors can be also used in an agent of the present invention.

The "compound having squalene synthase inhibitory activity" used in the present invention can be used in a form of a salt or a prodrug.

As for a "salt" of the compound having squalene synthase inhibitory activity used in the present invention, an acid addition salt, which is pharmaceutically and physiologically acceptable, is preferable. For such salts, for example, inorganic acids (e.g., hydrochloric acid, phosphoric acid, hydrobromic acid, sulfuric acid, etc.) or organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid, etc.) or the like are used. Further, in the case that "compound having squalene synthase inhibitory activity" used in the present invention has an acidic group such as carboxylic acid or the like, the "compound having squalene synthase inhibitory activity" may form salts with, for example, an inorganic base (e.g., an alkali metal or alkaline earth metal such as sodium, potassium, calcium, magnesium, or ammonia, etc.) or an organic base (e.g., tri-C₁₋₃ alkylamine such as triethylamine, etc.).

The "prodrug" of the compound having squalene synthase inhibitory activity [hereinafter, referred to as an "SSI Compound"] used in the present invention or a salt thereof refers to a compound which is converted to the SSI Compound by a reaction in vivo under the physiological condition with an enzyme, a gastric acid or the like, that is, a compound which is converted to the SSI Compound by enzymatic oxidation, reduction, hydrolysis, etc.; a compound which is converted to the SSI Compound by hydrolysis or the like with gastric acid, etc.; or the like. Examples of the prodrug of the SSI Compound include a compound wherein an amino group of the SSI Compound is substituted with acyl, alkyl or phosphoric acid (e.g.,, a compound wherein an amino group of the SSI Compound is substituted with eicosanoyl, alanyl, pentylaminocarbonyl, (5-methyl-2-oxo-1,3-dioxolen-4-yl)methoxycarbonyl, tetrahydrofuranyl, pyrrolidylmethyl, pivaloyloxymethyl or tert-butyl, etc.); a compound wherein a hydroxy group of the SSI Compound is substituted with acyl, alkyl, phosphoric acid or boric acid (e.g., a compound wherein a hydroxy group of the SSI Compound is substituted with acetyl, palmitoyl, propanoyl, pivaloyl, succinyl, fumaryl, alanyl or dimethylaminomethylcarbonyl, etc.); or a compound wherein a carboxyl group of the SSI Compound is substituted with ester or amide (e.g., a compound wherein a carboxyl group of the SSI Compound is substituted with ethyl ester, phenyl ester, carboxymethyl ester, dimethylaminomethyl ester, pivaloyloxymethyl ester, ethoxycarbonyloxyethyl ester, phthalidyl ester, (5-methyl-2-oxo-1,3-dioxolen-4-yl)methyl ester, cyclohexyloxycarbonylethyl ester or methyl amide, etc.); and the like. These compounds can be prepared from the SSI Compound by a per se known method.

In addition, the prodrug of the SSI Compound may be a compound which is converted into the SSI Compound under the physiological conditions as described in "Pharmaceutical Research and Development", Vol.7 (Molecular Design), pp. 163-198, published in 1990 by Hirokawa Publishing Co.

Further, the SSI Compound may be hydrated.

When the optically active form of the SSI Compound is needed, it can be obtained, for example, by using an optically active starting material, or by using a conventional method to optically resolve the racemic form of the SSI Compound. Further, the SSI Compound contains asymmetric carbon in its molecule, but when the compound has two stereoisomers of R-configuration and S-configuration, any isomer or a mixture thereof is included within the scope of the present invention.

In the formulae (I) and (Ia), examples of the hydrocarbon group in the "optionally substituted hydrocarbon group" represented by R₁ include an aliphatic chain (acyclic) hydrocarbon group, an alicyclic hydrocarbon group and an aryl group, with aliphatic chain hydrocarbon group being preferable.

The aliphatic chain hydrocarbon group of the hydrocarbon group includes a linear or branched aliphatic hydrocarbon group such as an alkyl group, an alkenyl group, an alkynyl group. Among these, the branched alkyl group is preferable. Examples of the alkyl group include C₁₋₇ alkyl such as methyl, ethyl, n-propyl, isopropyl, n-butyl, isobutyl, sec-butyl, tert-butyl, n-pentyl, isopentyl, neopentyl, 1-methylpropyl, n-hexyl, isohexyl, 1,1-dimethylbutyl, 2,2-dimethylbutyl, 3,3-dimethylbutyl, 3,3-dimethylpropyl, 2-ethylbutyl, n-heptyl and the like, with C₃₋₅ alkyl such as n-propyl, isopropyl, isobutyl, neopentyl and the like being preferable, and isobutyl, neopentyl and the like being particularly preferable. Examples of the alkenyl group include C₂₋₆ alkenyl such as vinyl, allyl, isopropenyl, 2-methylallyl, 1-propenyl, 2-methyl-l-propenyl, 2-methyl-2-propenyl, 1-butenyl, 2-butenyl, 3-butenyl, 2-ethyl-1-butenyl, 2-methyl-2-butenyl, 3-methyl-2-butenyl, 1-pentenyl, 2-pentenyl, 3-pentenyl, 4-pentenyl, 4-methyl-3-pentenyl, 1-hexenyl, 2-hexenyl, 3-hexenyl, 4-hexenyl, 5-hexenyl and the like, with vinyl, allyl, isopropenyl, 2-methylallyl, 2-methyl-1-propenyl, 2-methyl-2-propenyl, 3-methyl-2-butenyl and the like being particularly preferable. Examples of the alkynyl group include C₂₋₆ alkynyl such as ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl, 5-hexynyl and the like, with ethynyl, 1-propynyl, 2-propynyl being particularly preferable.

The alicyclic hydrocarbon group of the hydrocarbon group includes a saturated or unsaturated alicyclic hydrocarbon group such as a cycloalkyl group, a cycloalkenyl group, a cycloalkadienyl group and the like. As for the cycloalkyl group, a C₃₋₉ cycloalkyl group is preferable which includes, for example, cyclopropyl, cyclobutyl, cyclopentyl, cyclohexyl, cycloheptyl, cyclooctyl, cyclononyl and the like, with a C₃₋₆ cycloalkyl group such as cyclopropyl, cyclobutyl, cyclopentyl, cyclohexyl being preferable. Examples of the cycloalkenyl group include a C₅₋₆ cycloalkenyl group such as 2-cyclopenten-1-yl, 3-cyclopenten-1-yl, 2-cyclohexen-1-yl, 3-cyclohexene-1-yl, 1-cyclobuten-1-yl, 1-cyclopenten-1-yl and the like. Examples of the cycloalkadienyl group include a C₅₋₆ cycloalkadienyl group such as 2,4-cyclopentadien-1-yl, 2,4-cyclohexadien-1-yl, 2,5-cyclohexadien-1-yl.

The aryl group of the hydrocarbon group includes a C₆₋₁₆ monocyclic or fused polycyclic aromatic hydrocarbon group such as phenyl, naphthyl, anthryl, phenanthryl, acenaphthylenyl, with a C₆₋₁₀ aryl group such as phenyl, 1-naphthyl, 2-naphthyl being particularly preferable.

The substituent of the "optionally substituted hydrocarbon group" represented by R₁ includes, an optionally substituted aryl group, an optionally substituted cycloalkyl group, an optionally substituted cycloalkenyl group, an optionally substituted heterocyclic group, an optionally substituted amino group, an optionally substituted hydroxy group, an optionally substituted thiol group, a halogen atom (e.g., fluorine, chlorine, bromine, iodine) and oxo etc., and the hydrocarbon group is optionally substituted with 1 to 5 (preferably 1 to 3) arbitrary substituents at a substitutable position. Examples of the aryl group of the optionally substituted aryl group include a C₆₋₁₆ aryl group such as phenyl, naphthyl, anthryl, phenanthryl, acenaphthylenyl, with a C₆₋₁₀ aryl group such as phenyl, 1-naphthyl, 2-naphthyl being preferable. The substituent of the optionally substituted aryl group include a C₁₋₃ alkoxy group (e.g., methoxy, ethoxy, propoxy, etc.), a halogen atom (e.g., fluorine, chlorine, bromine, iodine, etc.), a C₁₋₃ alkyl group (e.g., methyl, ethyl, propyl, etc.), and the aryl group is optionally substituted with 1 to 2 arbitrary substituents. Examples of the cycloalkyl group of the optionally substituted cycloalkyl group include a C₃₋₇ cycloalkyl group such as cyclopropyl, cyclobutyl, cyclopentyl, cyclohexyl, cycloheptyl. The substituent and its substituted number of the optionally substituted cycloalkyl group are the same kind and number as those of the aforementioned substituent of optionally substituted aryl group. Examples of the cycloalkenyl group of the optionally substituted cycloalkenyl group include a C₃₋₆ cycloalkenyl group such as cyclopropenyl, cyclobutenyl, cyclopentenyl, cyclohexenyl. The substituent and its substituted number of the optionally substituted cycloalkenyl group are the same kind and number as those of the aforementioned substituent of optionally substituted aryl group. A heterocyclic group of the optionally substituted heterocyclic group includes an aromatic heterocyclic group and a saturated or unsaturated non-aromatic heterocyclic group (aliphatic heterocyclic group) containing at least one and preferably 1 to 4 hetero atoms selected from oxygen, sulfur and nitrogen as an atom constituting the ring system (ring atom) with an aromatic heterocyclic group being preferable. Examples of the aromatic heterocyclic group include a 5- to 6-membered aromatic monocyclic heterocyclic group (e.g., furyl, thienyl, pyrrolyl, oxazolyl, isoxazolyl, thiazolyl, isothiazolyl, imidazolyl, pyrazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pyridazinyl, pyrimidinyl, pyrazinyl, triazinyl, etc.) and an aromatic fused heterocyclic group in which 2 to 3 of 5-to 6-membered rings are fused (e.g., benzofuranyl, isobenzofuranyl, benzo[b]thienyl, indolyl, isoindolyl, 1H-indazolyl, benzimidazolyl, benzoxazolyl, 1,2-benzoisoxazolyl, benzothiazolyl, 1,2-benzoisothiazolyl, 1H-benzotriazolyl, quinolyl, isoquinolyl, cinnolyl, quinazolinyl, quinoxalinyl, phthalazinyl, naphthylizinyl, purinyl, pteridinyl, carbazolyl, α-carbolinyl, β-carbolinyl, γ-carbolinyl, acridinyl, phenoxazinyl, phenothiazinyl, phenazinyl, phenoxathinyl, thianthrenyl, phenoxathinyl, phenanthrolinyl, indolizinyl, pyrrolo[1,2-b]pyridazinyl, pyrazolo[1,5-a]pyridyl, imidazo[1,2-a]pyridyl, imidazo[1,5-a]pyridyl, imidazo[1,2-b]pyridazinyl, imidazo[1,2-a]pyrimidinyl, 1,2,9-triazolo[4,3-a]pyridyl, 1,2,4-triazolo[4,3-b]pyridazinyl, etc.), with the 5- to 6-membered aromatic monocyclic heterocyclic group such as furyl, thienyl, indolyl, isoindolyl, pyrazinyl, pyridyl, pyrimidinyl and the like being preferable. Examples of the non-aromatic heterocyclic group include a 4- to 8-membered non-aromatic heterocyclic group such as oxiranyl, azetidinyl, oxetanyl, thietanyl, pyrrolidinyl, tetrahydrofuryl, thiolanyl, piperidyl, tetrahydropyranyl, morpholinyl, thiomorpholinyl, piperazinyl. The optionally substituted heterocyclic group may contain 1 to 4, preferably 1 to 2 substituents, and these substituents include C₁₋₃ alkyl group (e.g., methyl, ethyl, propyl, etc.) and the like. The substituent in the optionally substituted amino group (including amino group, mono- or di-substituted amino group), an optionally substituted hydroxy group and an optionally substituted thiol group include, for example, lower (C₁₋₃) alkyl (e.g., methyl, ethyl, propyl, etc.). Further, when the hydrocarbon group in the optionally substituted hydrocarbon group represented by R₁ is an alicylcic hydrocarbon group or an aryl group, the substituent may be also a C₁₋₃ alkyl group (e.g., methyl, ethyl, propyl, etc.).

In addition, as described above, R₁ may contain an oxo group as a substituent, and R₁ may contain an acyl carboxylate group which is a hydrocarbon group substituted with oxo. Such examples include C₁₋₆ acyl group which may contain a substituent (e.g., formyl, acetyl, propionyl, butyryl, isobutyryl, valeryl, isovaleryl, pivaloyl, hexanoyl, dimethyl acetyl, trimethyl acetyl, etc.). Further, the acyl group may contain 1 to 5 substituent at a substitutable position, and the substituent includes a halogen atom (e.g., fluorine, chlorine, bromine).

In the formulae (I) and (Ia), the "optionally substituted hydrocarbon group" represented by R₂ and R₃ may include the group descried as the "optionally substituted hydrocarbon group" represented by R₁. However, an alkyl group, an aryl group and substituents thereof may be the group as follows. That is, as for the alkyl group of the "optionally substituted alkyl group" includes a C₁₋₆ lower alkyl group (e.g., methyl, ethyl, n-propyl, isopropyl, butyl, isobutyl, sec-butyl, tert-butyl, pentyl, isopentyl, neopentyl, hexyl, isohexyl, etc.), and preferably includes a C₁₋₄ alkyl group such as methyl, ethyl, propyl, isopropyl, butyl, tert-butyl. For example, these optionally substituted alkyl group may contain 1 to 4 substituents, and such substituents include a halogen atom (e.g., fluorine, chlorine, bromine, iodine), C₁₋₄ lower alkoxy group (e.g., methoxy, ethoxy, propoxy, isopropoxy, butoxy, tert-butoxy, etc.) and the like.

The "optionally substituted aryl group" includes monocyclic or fused polycyclic aromatic hydrocarbon group such as phenyl, naphthyl, anthryl, phenanthryl, acenaphthylenyl, with phenyl being particularly preferable. The substituent of the "optionally substituted aryl group" includes a halogen atom (e.g., fluorine, chlorine, bromine, iodine etc.), optionally substituted lower alkyl group, optionally substituted lower alkoxy group, an optionally substituted hydroxy group, nitro and cyano, and may be substituted with 1 to 3 (preferably 1 to 2) of such substituents of the same or different. Examples of the lower alkyl include a C₁₋₄ alkyl group such as methyl, ethyl, n-propyl, isopropyl, n-butyl, isobutyl, sec-butyl, tert-butyl, with methyl and ethyl being particularly preferable. Examples of the lower alkoxy include a C₁₋₄ alkoxy group such as methoxy, ethoxy, n-propoxy, isopropoxy, n-butoxy, isobutoxy, sec-butoxy, tert-butoxy, with methoxy and ethoxy being particularly preferable. The substituent of the optionally substituted lower alkyl and the optionally substituted lower alkoxy includes a halogen atom (e.g., fluorine, chlorine, bromine, iodine etc.), and may be 1 to 5 substituted at an arbitrary position. The substituent in the optionally substituted hydroxy group includes, for example, a lower (C₁₋₄) alkyl group (e.g., methyl, ethyl, propyl, isopropyl, butyl, tert-butyl etc.), a C₃₋₆ cycloalkyl group (e.g., cyclopropyl, cyclobutyl, cyclopentyl, cyclohexyl, etc.), a C₆₋₁₀ aryl group (e.g., phenyl, 1-naphthyl, 2-naphthyl, etc.), a C₇₋₁₂ aralkyl group (e.g., benzyl, phenethyl, etc.). Further, these substituents may be taken together with the adjacent substituents to form a ring; a group represented by 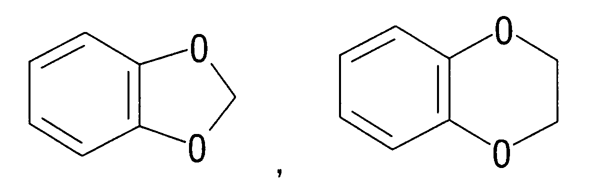 may be used when the aryl group of the "optionally substituted aryl group" represented by R₂ and R₃ is a phenyl group; and may be substituted with 1 to 4 substituents such as lower (C₁₋₃) alkyl group (e.g., methyl, ethyl, propyl, isopropyl, etc.).

The heterocyclic group of the "optionally substituted heterocyclic group" represented by R₂ and R₃ include a heterocyclic group described in detail for the "optionally substituted heterocyclic group" given as a substituent of the "optionally substituted hydrocarbon group" represented by R₁. Among those, 5- to 6-membered aromatic monocyclic heterocyclic ring such as furyl, thienyl, indolyl, isoindolyl, pyrazinyl, pyridyl, pyrimidyl, imidazolyl or the like is particularly preferable. The substituent of the heterocyclic group includes C₁₋₃ alkyl (e.g., methyl, ethyl, propyl, etc.), and said heterocyclic ring may have 1 to 4 of such substituents.

As described above, as for R₂ and R₃, an optionally substituted phenyl group is preferable, and more preferably, the substituted phenyl group, especially, a phenyl group substituted with 1 to 3, preferably 1 to 2 of a halogen atom such as chlorine and bromine, lower (C₁₋₃) alkoxy or the like is preferable. Further, it is preferable in that any one of R₂ and R₃ is a hydrogen atom.

In the formula (I), the "substituent comprising an optionally esterified carboxyl group" represented by X' includes an optionally esterified carboxyl group, and a group containing optionally esterified carboxyl group. The optionally esterified carboxyl group includes the same group as that defined with respect to Y hereinafter.

The "substituent comprising an optionally substituted carbamoyl group" represented by X' includes an optionally substituted carbamoyl group, and a group containing an optionally substituted carbamoyl group. The optionally substituted carbamoyl group includes the same group as that define with respect to Y hereinafter.

The "substituent comprising an optionally substituted hydroxy group" represented by X' includes an optionally substituted hydroxy group, and a group containing an optionally substituted hydroxy group. The optionally substituted hydroxy group includes the same group as that defined with respect to Y hereinafter.

The "substituent comprising an optionally substituted amino group" represented by X' includes an optionally substituted amino group, and a group containing an optionally substituted amino group. The optionally substituted amino group includes the same group as that defined with respect to Y hereinafter.

The "substituent comprising an optionally substituted heterocyclic residue having a hydrogen which can be deprotonated" represented by X' includes an optionally substituted heterocyclic residue having a hydrogen atom which can be deprotonated (i.e., having an active proton), and a group containing an optionally substituted heterocyclic residue having a hydrogen atom which can be deprotonated. The optionally substituted heterocyclic residue having a hydrogen atom which can be deprotonated includes the same group as that defined with respect to Y hereinafter.

X' includes a group represented by the formula (a): 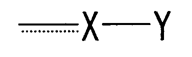 wherein, X is a bond, or divalent or trivalent atomic chain, Y is an optionally esterified carboxyl group, an optionally substituted carbamoyl group, an optionally substituted hydroxy group, an optionally substituted amino group, or an optionally substituted heterocyclic residue having a hydrogen atom which can be deprotonated, and the dotted line is a single or double bond.

In the formula (a), the "divalent atomic chain" represented by X may be any divalent chain having preferably 1 to 7 and more preferably 1 to 4 of atoms composing the linear portion, and may contain a side chain.

Example thereof includes a group represented by 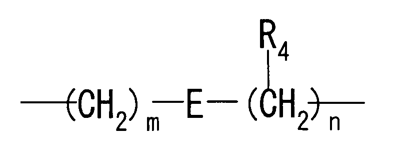 wherein, m and n is an integer of 0, 1, 2 or 3, independently, E is a bond or an oxygen atom, a sulfur atom, sulfoxide, sulfone, -N (R₅)-, -NHCO-, -CON(R₆)- or -NHCONH-. Herein, R₄ and R₆ represent a hydrogen atom, an optionally substituted lower alkyl group, an optionally substituted aralkyl group or an optionally substituted phenyl group. In addition, R₅ represents a hydrogen atom, a lower alkyl group, an aralkyl group or an acyl group.

The alkyl group of the "optionally substituted lower alkyl group" represented by R₄ and R₆ includes a C₁₋₆ linear or branched lower alkyl group (e.g., methyl, ethyl, n-propyl, isopropyl, n-butyl, isobutyl, tert-butyl, n-pentyl, isopentyl, neopentyl, etc.). The optionally substituted lower alkyl group may have 1 to 4, preferably 1 to 2 substituents, and such substituents include an aromatic heterocyclic group (e.g., 5- to 6-membered aromatic heterocyclic ring containing 1 to 4 hetero atoms of N, O, S such as furyl, thienyl, indolyl, isoindolyl, pyrazinyl, pyridyl, pyrimidyl, imidazolyl, etc.), an optionally substituted amino group, an optionally substituted hydroxy group, an optionally substituted thiol group, an optionally esterified carboxyl group and a halogen atom (e.g., fluorine, chlorine, bromine, iodine). The substituent in the optionally substituted amino group (including amino group, mono- or di-substituted amino group), an optionally substituted hydroxy group and an optionally substituted thiol group include, for example, lower (C₁₋₃) alkyl (e.g., methyl, ethyl, propyl, etc.). Examples of the optionally esterified carboxyl group include C₂₋₅ alkoxycarbonyl such as methoxycarbonyl ethoxycarbonyl, propoxycarbonyl, phenoxycarbonyl, 1-naphthoxycarbonyl etc. and C₇₋₁₁ aryloxycarbonyl, with methoxycarbonyl, ethoxycarbonyl, propoxycarbonyl being preferable.

The aralkyl group of the "optionally substituted aralkyl group" represented by R₄ and R₆ includes a C₇-C₁₅ aralkyl group such as benzyl, naphthylmethyl, phenylpropyl, phenylbutyl. The optionally substituted aralkyl group may have 1 to 4, preferably 1 to 2 substituents, and such substituents include a halogen atom (e.g., fluorine, chlorine, bromine, iodine), a C₁₋₃ alkoxy group (e.g., methoxy, ethoxy, propoxy group), a hydroxy group, an amino group, a carboxyl group, a sulfhydryl group.

The substituent of the "optionally substituted phenyl group" represented by R₄ and R₆ includes a halogen atom (e.g., fluorine, chlorine, bromine, iodine), a C₁₋₃ alkoxy (e.g., methoxy, ethoxy, propoxy, etc.), C₁₋₃ alkyl (e.g., methyl, ethyl, propyl).

Provided that, R₄ may be different in every methylene chain.

In addition, examples of the "lower alkyl group" and the "aralkyl group" represented by R₅ include a C₁₋₄ lower alkyl group (e.g., methyl, ethyl, propyl, butyl, tert-butyl, etc.), a C₇₋₁₅ aralkyl group (e.g., benzyl, phenethyl, phenylpropyl, phenylbutyl, naphthylmethyl, etc.), respectively.

Examples of the "acyl group" represented by R₅ include a lower (C₁₋₆) alkanoyl group (e.g., formyl, acetyl, propionyl, butyryl, isobutyryl, valeryl, isovaleryl, pivaloyl, hexanoyl, etc.), a lower (C₃₋₇) alkenoyl group (e.g., acryloyl, methacryloyl, crotonoyl, isocrotonoyl, etc.), a C₄₋₇ cycloalkane carbonyl group (e.g., a cyclopropane carbonyl group, a cyclobutane carbonyl group, a cyclopentane carbonyl group, a cyclohexane carbonyl group, etc.), a lower (C₁₋₄) alkane sulfonyl group (e.g., mesyl, ethane sulfonyl, propane sulfonyl, etc.), a C₇₋₁₄ aroyl group (e.g., benzoyl, p-toluoyl, 1-naphthoyl, 2-naphthoyl, etc.), a C₆₋₁₀ aryl lower (C₂₋₄) alkanoyl group (e.g., phenylacetyl, phenylpropionyl, hydroatropoyl , phenylbutyryl, etc.), a C₆₋₁₀ aryl lower (C₃₋₅) alkenoyl group (e.g., cinnamoyl, atropoyl, etc.), a C₆₋₁₀ arenesulfonyl group (e.g., benzensulfonyl, a p-toluenesulfonyl group, etc.).

Further, X may be a carbon chain having a double bond or -L-CH(OH)- (L is a bond or a linear or branched alkylene chain). The "carbon chain having a double bond" may have. 1 to 7 and more preferably 1 to 4 of carbon atoms constituting the linear portion, and may also contain a side chain. The double bond in the carbon chain is contained in any one or both of a linear portion and a branched portion, with preferably contained in the linear portion. Further, the number of double bonds contained in the carbon chain is not particularly limited, if possible, but 1 to 2 is preferable.

Examples of the carbon chain containing double bond include methine, vinylene, propenylene, butenylene, butadienylene, methylpropenylene, ethylpropenylene, propylpropenylene, methylbutenylene, ethylbutenylene, propylbutenylene, methylbutadienylene, ethylbutadienylene, propylbutadienylene, pentenylene, hexenylene, heptenylene, pentadienylene, hexadienylene, heptadienylene, with methine, vinylene, propenylene, butenylene, butadienylene being preferable. Herein, when the carbon chain is trivalent, the carbon chain forms a double bond with a substitutable carbon atom on the ring of ring J'.

Examples of the "linear or branched alkylene chain" represented by L include a linear or branched C₁₋₆ alkylene chain, for example, a divalent group such as methylene, ethylene, trimethylene, tetramethylene, pentamethylene, hexamethylene, heptamethylene, propylene, ethylmethylene, ethylethylene, propylethylene, butylethylene, methyltetramethylene, methyltrimethylene with a C₁₋₃ chain such as methylene, ethylene, trimethylene, propylene being preferable.

Among these, X' is preferably a group represented by the formula (b):

―X₁―Y

wherein X₁ is a bond or divalent atomic chain, Y is an optionally esterified carboxyl group, an optionally substituted carbamoyl group, an optionally substituted hydroxy group, an optionally substituted amino group or an optionally substituted heterocyclic group having a hydrogen which can be deprotonated.

In the formula (b), the divalent atomic chain represented by X₁ is the divalent atomic chain as defined as X above in the same manner.

In the formulae (a) and (b), the "divalent atomic chain" represented by X or X₁ may be a linear or branched alkylene chain having 1 to 7 (more preferably 1 to 4) of carbon atoms constituting the linear portion. Examples of the alkylene chain include a divalent group such as methylene, ethylene, trimethylene, tetramethylene, pentamethylene, hexamethylene, heptamethylene, propylene, ethylmethylene, ethylethylene, propylethylene, butylethylene, methyltetramethylene, methyltrimethylene, with a C₁₋₄ chain such as methylene, ethylene, trimethylene, propylene being preferable.

In the formulae (a) and (b), the "optionally esterified carboxyl group" represented by Y includes a C₂₋₇ lower alkoxycarbonyl (e.g., methoxycarbonyl, ethoxycarbonyl, propoxy carbonyl, isopropoxycarbonyl, butoxycarbonyl, tert-butoxycarbonyl, sec-butoxycarbonyl, pentyloxycarbonyl, isopentyloxycarbonyl, neopentyloxycarbonyl, etc.), C₇₋₁₄ aryloxycarbonyl (e.g., phenoxycarbonyl, 1-naphthoxycarbonyl), C₈₋₁₂ aralkyloxycarbonyl (e.g., benzyloxycarbonyl, etc.). Among these, a carboxyl group, methoxycarbonyl, and ethoxycarbonyl are preferable.

The substituent of the "optionally substituted carbamoyl group" represented by Y includes optionally substituted lower (C₁₋₆) alkyl (e.g., methyl, ethyl, n-propyl, isopropyl, butyl, isobutyl, sec-butyl, tert-butyl, pentyl, isopentyl, neopentyl, hexyl, isohexyl, etc.), optionally substituted C₃₋₆ cycloalkyl (e.g., cyclopropyl, cyclobutyl, cyclopentyl, cyclohexyl, etc.), optionally substituted C₆₋₁₄ aryl group (e.g., phenyl, 1-naphthyl, 2-naphthyl, etc.), an optionally substituted C₇₋₁₁ aralkyl group (e.g., benzyl, phenethyl, etc.), and may be substituted, the same or different, with 1 to 2 substituents. The substituent in the optionally substituted lower (C₁₋₆) alkyl and optionally substituted C₃₋₆ cycloalkyl includes carboxyl group optionally esterified with lower (C₁₋₅) alkyl (e.g., methyl, ethyl, propyl, isopropyl, butyl, tert-butyl, pentyl, isopentyl, neopentyl), a 5- to 6-membered aromatic heterocyclic ring group containing 1 to 4 hetero atoms (e.g., furyl, thienyl, indolyl, isoindolyl, pyrazinyl, pyridyl, pyrimidyl, imidazolyl, etc.), an amino group, a hydroxy group and a phenyl group, and such substituents may be 1 to 3 substituted of the same or different substituents. The substituent of the optionally substituted aryl group and the optionally substituted aralkyl group includes a halogen atom (e.g., fluorine, chlorine, bromine, iodine), and carboxyl group optionally esterified with a lower (C₁₋₄) alkyl group (e.g., methyl, ethyl, propyl, isopropyl, butyl, tert-butyl, etc.). In addition, as for the optionally substituted carbamoyl group, the two substituents on the nitrogen atoms may be taken together with the nitrogen atoms to form a cyclic amino group, and such cyclic amino groups include, for example, 1-azetidinyl, 1-pyrrolidinyl, piperidino, morpholino, 1-piperazinyl. Further, the cyclic amino group may also contain a substituent.

The substituent of the "optionally substituted hydroxy group" represented by Y includes, for example, lower (C₁₋₄) alkyl (e.g., methyl, ethyl, propyl, isopropyl, butyl, tert-butyl, etc.), a C₃₋₆ cycloalkyl group (e.g., cyclopropyl, cyclobutyl, cyclopentyl, cyclohexyl, etc.), an optionally substituted C₆₋₁₀ aryl group (e.g., phenyl, 1-naphthyl, 2-naphthyl, etc.), an optionally substituted C₇₋₁₁ aralkyl group (e.g., benzyl, phenethyl, etc.). The substituent of the optionally substituted aryl group and the optionally substituted aralkyl group includes a halogen atom (e.g., fluorine, chlorine, bromine, iodine), carboxyl group optionally esterified with a lower (C₁₋₄) alkyl group (e.g., methyl, ethyl, propyl, isopropyl, butyl, tert-butyl, etc.), and the like.

The "optionally substituted amino group" represented by Y includes a mono- and di-substituted amino group, and examples of such substituent include lower (C₁₋₄) alkyl (e.g., methyl, ethyl, propyl, isopropyl, butyl, tert-butyl, etc.), a C₃₋₆ cycloalkyl group (e.g., cyclopropyl, cyclobutyl, cyclopentyl, cyclohexyl, etc.), an optionally substituted C₆₋₁₀ aryl group (e.g., phenyl , 1-naphthyl, 2-naphthyl, etc.), and an optionally substituted C₋₇₋₁₁ aralkyl group (e.g., benzyl, phenethyl, etc.). The substituent of the optionally substituted aryl group and the optionally substituted aralkyl group includes a halogen atom (e.g., fluorine, chlorine, bromine, iodine), carboxyl group optionally esterified with a lower (C₁₋₄) alkyl group (e.g., methyl, ethyl, propyl, isopropyl, butyl, tert-butyl, etc.), and the like, and these groups may have 1 to 4 and preferably 1 to 2 substituents. In addition, the two substituents on the nitrogen atoms may be taken together with the nitrogen atom to form a cyclic amino group, and such cyclic amino groups include, for example, 1-azetidinyl, 1-pyrrolidinyl, piperidino, morpholino, 1-piperazinyl. Further, the cyclic amino group may also contain a substituent.

The heterocyclic residue of the "optionally substituted heterocyclic group having a hydrogen atom which can be deprotonated" represented by Y includes a 5- to 7-membered (preferably 5-membered) monocyclic heterocyclic residue having at least one selected from N, S, O (preferably a nitrogen-containing heterocyclic residue), and may contain a hydrogen atom that may form a proton by elimination thereof. Examples include tetrazol-5-yl or a group represented by the formula: 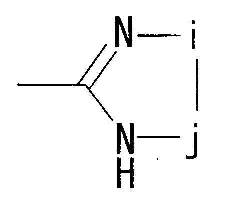 wherein, i is -O- or -S-, j is >C=O, >C=S or >S(O)₂ (among these, 2,5-dihydro-5-oxo-1,2,4-oxadiazol-3-yl, 2,5-dihydro-5-thioxo-1,2,4-oxadiazol-3-yl, 2,5-dihydro-5-oxo-1, 2,4-thiadiazol-3-yl are preferable).

The heterocyclic residue may be protected with an optionally substituted lower alkyl group (preferably C₁₋₄ alkyl) or an acyl group. Examples of the optionally substituted lower alkyl group include C₁₋₄ alkyl optionally substituted with 1) phenyl optionally substituted with C₁₋₃ alkyl, nitro or C₁₋₃ alkoxy or 2) C₁₋₃ alkoxy (methyl, triphenylmethyl, methoxymethyl, ethoxymethyl, p-methoxybenzyl, p-nitrobenzyl, etc.). Examples of the acyl group include lower (C₂₋₅) alkanoyl, benzoyl and the like.

Among these, X' is preferably an alkyl group substituted with an optionally esterified carboxyl group, an alkyl group substituted with an optionally substituted heterocyclic residue having a hydrogen which can be deprotonated or an alkyl group substituted with an optionally substituted carbamoyl group.

In the formula (I), the heterocyclic ring represented by Ring A includes a heterocyclic group described in detail with respect to the substituent of the hydrocarbon group represented by R₁. Among them, a group represented below is preferable.

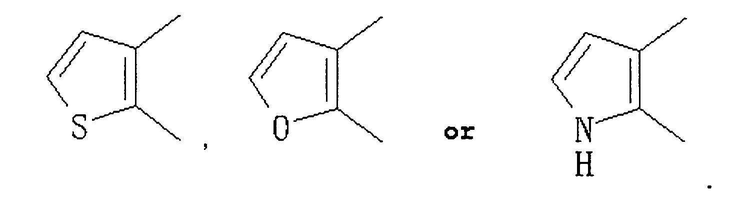

The substituent of the "optionally substituted benzene ring" and "optionally substituted heterocyclic ring" represented by Ring A includes a halogen atom (e.g., fluorine, chlorine, bromine, iodine), an optionally substituted C₁₋₄ lower alkyl group (e.g., methyl, ethyl, propyl, butyl, tert-butyl, etc.), an optionally substituted C₁₋₄ lower alkoxy group (e.g., methoxy, ethoxy, propoxy, isopropoxy, butoxy, tert-butoxy, etc.), a hydroxy group, a nitro group and cyano. Ring A may have 1 to 3 and preferably. 1 to 2 said substituents. Further, these substituents may be taken together with the adjacent substituents to form a ring. The substituent of the optionally substituted lower alkyl group and the optionally substituted lower alkoxy group includes a halogen atom (e.g., fluorine, chlorine, bromine, iodine), in which 1 to 3 substituents may be present at an arbitrary position. Ring A substituted with methoxy or a chlorine atom is preferable, and Ring A substituted with a chlorine atom is particularly preferable.

In the formula (Ia), the substituent of the "optionally substituted benzene ring" represented by the Ring B includes a halogen atom (e.g., fluorine, chlorine, bromine, iodine), an optionally substituted C₁₋₄ lower alkyl group (e.g., methyl, ethyl, propyl, butyl, tert-butyl, etc.), an optionally substituted C₁₋₄ lower alkoxy group (e.g., methoxy, ethoxy, propoxy, isopropoxy, butoxy, tert-butoxy, etc.), a hydroxy group, a nitro group and cyano. Ring B may have 1 to 3 and preferably 1 to 2 said substituents. Further, these substituents may be taken together with the adjacent substituents to form a ring. The substituent of the optionally substituted lower alkyl group and the optionally substituted lower alkoxy group includes a halogen atom (e.g., fluorine, chlorine, bromine, iodine), in which 1 to 3 substituents may be present at an arbitrary position. Ring B substituted with methoxy or a chlorine atom is preferable, and Ring B substituted with a chlorine atom is particularly preferable.

In the formula (I), the heterocyclic ring in the "7-to 8-membered heterocyclic ring containing 3 or less hetero atoms as atoms constituting a ring" represented by the ring J' includes, for example, a saturated or unsaturated 7- to 8-membered heterocyclic ring containing at least one selected from O, S(O)_{q} (q is an integer of 0, 1 or 2) and N. Within the atoms constituting the ring (ring constituting atom) of the heterocyclic ring, there are three or less hetero atoms.

Further, Ring J', in addition to a group represented by R₁, R₂, R₃ and X', may have 1 to 2 substituent at a substitutable position. Examples of the substituent, when the substituent is attached to a nitrogen atom on Ring J', include an alkyl group (e.g., C₁₋₆ alkyl such as methyl, ethyl, n-propyl, isopropyl, n-butyl, isobutyl, tert-butyl, n-pentyl, isopentyl, neopentyl, etc.), an acyl group (e.g., C₁₋₄ acyl group such as formyl, acetyl, propionyl, butyroyl, etc.). The alkyl group or acyl group may further be substituted with 1 to 5 halogen atom (e.g., fluorine, chlorine, bromine, iodine). Further, when the substituent is attached to a carbon atom on the Ring J', examples of the substituent include oxo, thioxo, an optionally substituted hydroxy group and an optionally substituted amino group. As for the optionally substituted hydroxy group and the optionally substituted amino group, the "optionally substituted hydroxy group" and the "optionally substituted amino group" defined as Y above is mentioned in the same manner.

As for Ring J', it is preferable to have oxo or thioxo substituted on a substitutable position, in addition to the group represented by R₁, R₂, R₃ and X'.

Examples of a fused ring with Ring A and the ring J' include

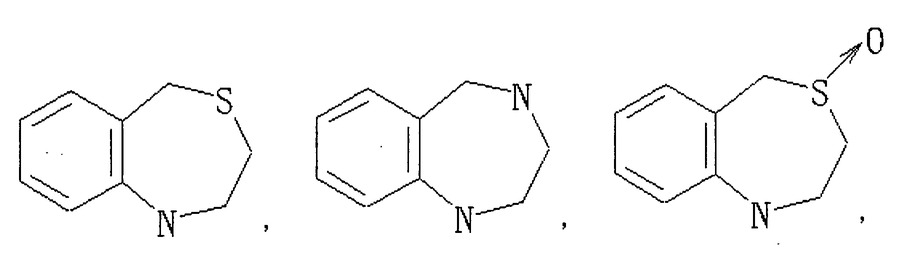

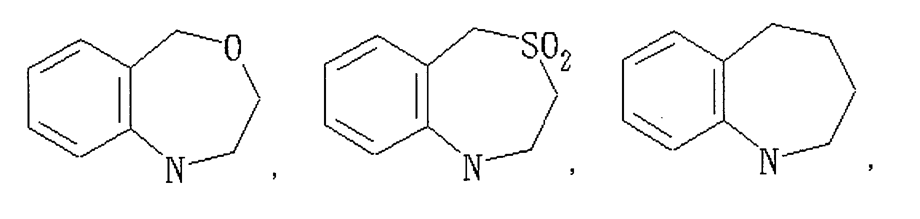

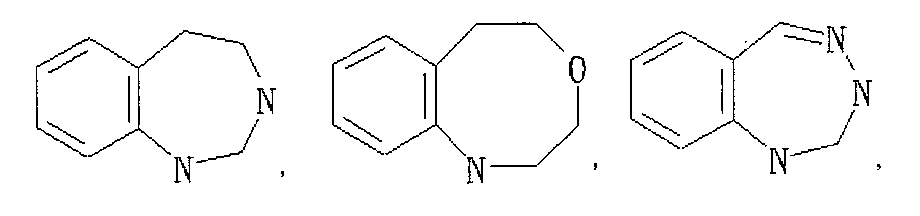

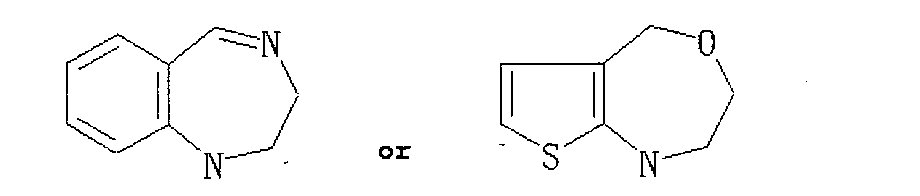

The formula (I) is preferably a group represented by the formula (I') 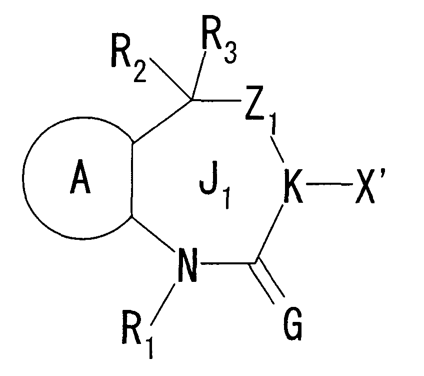 wherein, R₁, R₂, R₃, X' and Ring A is as defined above. Ring J₁ is a 7-membered heterocyclic ring, Z₁ is -N(R₇)- (R₇ is a hydrogen atom, an alkyl group or an acyl group), -S(O)_{q} (q is an integer of 0, 1 or 2), -CH₂- or -O-, K is C or N, and G is O or S.

In the formula (I') above, the alkyl group represented by R₇ includes a C₁₋₆ linear or branched lower alkyl group (e.g., methyl, ethyl, n-propyl, isopropyl, n-butyl, isobutyl, tert-butyl, n-pentyl, isopentyl, neopentyl, etc.), and may be substituted with 1 to 5 halogen atom (e.g., fluorine, chlorine, bromine, iodine).

Examples of the acyl group represented by R₇ include a C₁₋₄ acyl group (e.g., formyl, acetyl, propionyl, butyroyl, etc.), and which may be substituted with 1 to 5 halogen atom (e.g., fluorine, chlorine, bromine, iodine).

In the formula (I'), Z₁ is preferably S(O)_{q} (q is an integer of 0, 1 or 2) or O. Further, K is preferably C and G is preferably O.

The formula (I') is more preferably a compound represented by the formula (I'') 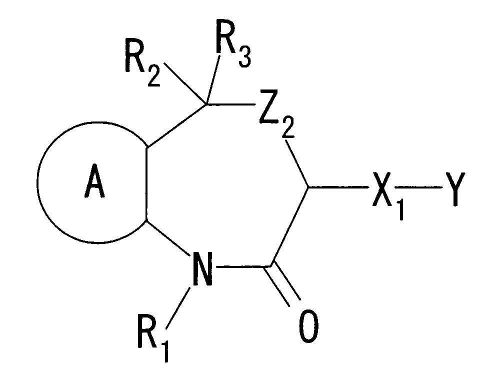 wherein, R₁, R₂, R₃, X₁, Y and Ring A is as defined above. Z₂ is S(O)_{q} (q is an integer of 0, 1 or 2) or O.

The compound represented by the formula (I) is preferably the compound represented by the formula (Ia)

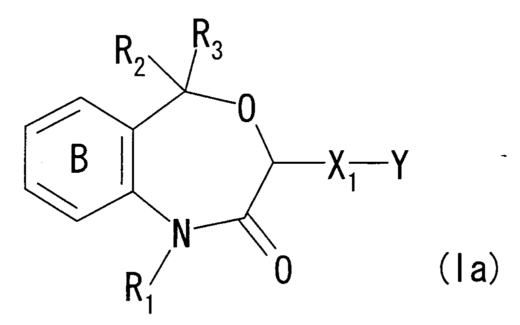

The formula (Ia) may be also a compound represented by the formula (Ia') 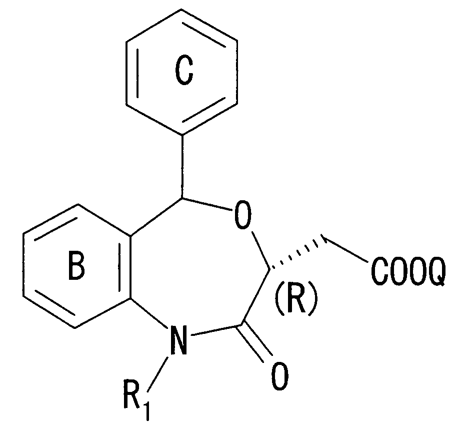 wherein, R₁ and Ring B are as defined above. Q is a hydrogen atom or a metal ion, Ring C is an optionally substituted phenyl group. In the formula, the substituents at 3- and 5-position represent trans which faces the opposite direction relative to the surface of the 7-membered ring, and (R) represents R-configuration.

In the formula (Ia'), the metal ion represented by Q includes a sodium ion, a potassium ion, a calcium ion, an aluminum ion, with a sodium ion and a potassium ion being preferable.

The substituent of the "optionally substituted phenyl group" represented by Ring C includes the same group as the substituent of the "optionally substituted aryl group" described as an example of the "optionally substituted hydrocarbon group" defined with respect to R₂ and R₃ above.

Examples of the salt of the compound represented by the formula (I), which are pharmacologically acceptable, include an inorganic salt such as hydrochloride, hydrobromic acid salt, sulfate, nitrate and phosphate, an organic acid salt such as acetate, tartrate, citrate, fumaric acid salt, maleate, toluenesulfonate and methanesulfonate, a metal salt such as a sodium salt, a potassium salt, a calcium salt and an aluminum salt, and a basic salt such as triethylamine salt, guanidine salt, ammonium salt, hydrazine salt, quinine salt and cinchonine salt. Among these, a sodium salt is preferable.

Specific examples of the compound represented by the formula (I) includes below: (3R,5S)-7-cyano-5-(2,3-dimethoxyphenyl)-1-neopentyl-2-oxo-1,2,3,5-tetrahydro-4,1-benzoxazepine-3-acetic acid, (3R,5S)-7-cyano-5-(2,4-dimethoxyphenyl)-1-neopentyl-2-oxo-1,2,3,5-tetrahydro-4,1-benzoxazepine-3-acetic acid, (3R,5S)-7-cyano-5-(2,3-methylenedioxyphenyl)-1-neopentyl-2-oxo-1,2,3,5-tetrahydro-4,1-benzoxazepine-3-acetic acid, (3R,5S)-7-cyano-5-(2,3-ethylenedioxyphenyl)-1-neopentyl-2-oxo-1,2,3,5-tetrahydro-4,1-benzoxazepine-3-acetic acid, (3R,5S)-7-cyano-5-(2,3-dimethoxyphenyl)-1-isobutyl-2-oxo-1,2,3,5-tetrahydro-4,1-benzoxazepine-3-acetic acid, (3R,5S)-7-cyano-5-(2,4-dimethoxyphenyl)-1-isobutyl-2-oxo-1,2,3,5-tetrahydro-4,1-benzoxazepine-3-acetic acid, (3R,5S)-7-cyano-5-(2,3-methylenedioxyphenyl)-1-isobutyl-2-oxo-1,2,3,5-tetrahydro-4,1-benzoxazepine-3-acetic acid, (3R,5S)-7-cyano-5-(2,3-ethylenedioxyphenyl)-1-isobutyl-2-oxo-1,2,3,5-tetrahydro-4,1-benzoxazepine-3-acetic acid, (3R,5S)-7-chloro-5-(2,3-dimethoxyphenyl)-1-neopentyl-2-oxo-1,2,3,5-tetrahydro-4,1-benzoxazepine-3-acetic acid, (3R,5S)-7-chloro-5-(2,4-dimethoxyphenyl)-1-neopentyl-2-oxo-1,2,3,5-tetrahydro-4,1-benzoxazepine-3-acetic acid, (3R,5S)-7-chloro-5-(2,3-methylenedioxyphenyl)-1-neopentyl-2-oxo-1,2,3,5-tetrahydro-4,1-benzoxazepine-3-acetic acid, (3R,5S)-7-chloro-5-(2,3-ethylenedioxyphenyl)-1-neopentyl-2-oxo-1,2,3,5-tetrahydro-4,1-benzoxazepine-3-acetic acid, (3R,5S)-7-chloro-5-(2,3-dimethoxyphenyl)-1-isobutyl-2-oxo-1,2,3,5-tetrahydro-4,1-benzoxazepine-3-acetic acid, (3R,5S)-7-chloro-5-(2,4-dimethoxyphenyl)-1-isobutyl-2-oxo-1,2,3,5-tetrahydro-9,1-benzoxazepine-3-acetic acid, (3R,5S)-7-chloro-5-(2,3-methylenedioxyphenyl)-1-isobutyl-2-oxo-1,2,3,5-tetrahydro-4,1-benzoxazepine-3-acetic acid, (3R,5S)-7-chloro-5-(2,3-ethylenedioxyphenyl)-1-isobutyl-2-oxo-1,2,3,5-tetrahydro-4,1-benzoxazepine-3-acetic acid, (3R,5S)-7-cyano-5-(2,3-dimethoxyphenyl)-1-neopentyl-2-oxo-1,2,3,5-tetrahyd,ro-4,1-benzothiazepine-3-acetic acid, (3R,5S)-7-cyano-5-(2,4-dimethoxyphenyl)-1-neopentyl-2-oxo-1,2,3,5-tetrahydro-4,1-benzothiazepine-3-acetic acid, (3R,5S)-7-cyano-5-(2,3-methylenedioxyphenyl)-1-neopentyl-2-oxo-1,2,3,5-tetrahydro-4,1-benzothiazepine-3-acetic acid, (3R,5S)-7-cyano-5-(2,3-ethylenedioxyphenyl)-1-neopentyl-2-oxo-1,2,3,5-tetrahydro-4,1-benzothiazepine-3-acetic acid, (3R,5S)-7-cyano-5-(2,3-dimethoxyphenyl)-1-isobutyl-2-oxo-1,2,3,5-tetrahydro-4,1-benzothiazepine-3-acetic acid, (3R,5S)-7-cyano-5-(2,4-dimethoxyphenyl)-1-isobutyl-2-oxo-1,2,3,5-tetrahydro-4,1-benzothiazepine-3-acetic acid, (3R,5S)-7-cyano-5-(2,3-methylenedioxyphenyl)-1-isobutyl-2-oxo-1,2,3,5-tetrahydro-4,1-benzothiazepine-3-acetic acid, (3R,5S)-7-cyano-5-(2,3-ethylenedioxyphenyl)-1-isobutyl-2-oxo-1,2,3,5-tetrahydro-4,1-benzothiazepine-3-acetic acid, (3R,5S)-7-chloro-5-(2,3-dimethoxyphenyl)-1-neopentyl-2-oxo-1,2,3,5-tetrahydro-4,1-benzothiazepine-3-acetic acid, (3R,5S)-7-chloro-5-(2,4-dimethoxyphenyl)-1-neopentyl-2-oxo-1,2,3,5-tetrahydro-4,1-benzothiazepine-3-acetic acid, (3R,5S)-7-chloro-5-(2,3-methylenedioxyphenyl)-1-neopentyl-2-oxo-1,2,3,5-tetrahydro-4,1-benzothiazepine-3-acetic acid, (3R,5S)-7-chloro-5-(2,3-ethylenedioxyphenyl)-1-neopentyl-2-oxo-1,2,3,5-tetrahydro-4,1-benzothiazepine-3-acetic acid, (3R,5S)-7-chloro-5-(2,3-dimethoxyphenyl)-1-isobutyl-2-oxo-1,2,3,5-tetrahydro-4,1-benzothiazepine-3-acetic acid, (3R,5S)-7-chloro-5-(2,4-dimethoxyphenyl)-1-isobutyl-2-oxo-1,2,3,5-tetrahydro-4,1-benzothiazepine-3-acetic acid, (3R,5S)-7-chloro-5-(2,3-methylenedioxyphenyl)-1-isobutyl-2-oxo-1,2,3,5-tetrahydro-4,1-benzothiazepine-3-acetic acid, (3R,5S)-7-chloro-5-(2,3-ethylenedioxyphenyl)-1-isobutyl-2-oxo-1,2,3,5-tetrahydro-4,1-benzothiazepine-3-acetic acid, (3R,5S)-7-cyano-5-(2-chlorophenyl)-1-neopentyl-2-oxo-1,2,3,5-tetrahydro-4,1-benzoxazepine-3-acetic acid, (3R,5S)-7-cyano-5-(2-chlorophenyl)-1-isobutyl-2-oxo-1,2,3,5-tetrahydro-4,1-benzoxazepin-3-acetic acid, (3R,5S)-7-chloro-5-(2-chlorophenyl)-1-neopentyl-2-oxo-1,2,3,5-tetrahydro-4,1-benzoxazepine-3-acetic acid, (3R,5S)-7-chloro-5-(2-chlorophenyl)-1-isobutyl-2-oxo-1,2,3,5-tetrahydro-4,1-benzoxazepine-3-acetic acid, (3R,5S)-7-cyano-5-(2-chlorophenyl)-1-neopentyl-2-oxo-1,2,3,5-tetrahydro-4,1-benzothiazepine-3-acetic acid, (3R,5S)-7-cyano-5-(2-chlorophenyl)-1-isobutyl-2-oxo-1,2,3,5-tetrahydro-4,1-benzothiazepine-3-acetic acid, (3R,5S)-7-chloro-5-(2-chlorophenyl)-1-neopentyl-2-oxo-1,2,3,5-tetrahydro-4,1-benzothiazepine-3-acetic acid, (3R,5S)-7-chloro-5-(2-chlorophenyl)-1-isobutyl-2-oxo-1,2,3,5-tetrahydro-4,1-benzothiazepine-3-acetic acid, (3R,5S)-7-chloro-5-(4-ethoxy-2-methoxyphenyl)-1-neopentyl-2-oxo-1,2,3,5-tetrahydro-4,1-benzoxazepine-3-acetic acid, (3R)-7-chloro-5-(2-chlorophenyl)-2,3-dihydro-1-isobutyl-2-oxo-1H-1,4-benzodiazepine-3-acetic acid, (3R,5S)-7-chloro-5-(2-chlorophenyl)-2,3,4,5-tetrahydro-1-isobutyl-2-oxo-1H-1,4-benzodiazepine-3-acetic acid, N-[[(3R,5S)-7-chloro-5-(2-chlorophenyl)-1-neopentyl-2-oxo-1,2,3,5-tetrahydro-4,1-benzothiazepine-3-yl]-acetyl]glycin, (3R,5S)-7-chloro-5-(2-chlorophenyl)-3-dimethylaminocarbonylmethyl-1-neopentyl-2-oxo-1,2,3,5-tetrahydro-4,1-benzothiazepine, 7-chloro-5-(2-chlorophenyl)-1-isobutyl-2-oxo-2,3,4,5-tetrahydro-1H-[1]-benzazepine-3-acetic acid, (3R,5S)-7-chloro-5-(2-chlorophenyl)-1-neopentyl-1,2,3,5-tetrahydro-2-thioxo-4,1-benzoxazepine-3-acetic acid, (3R,5S)-7-chloro-5-(2-chlorophenyl)-1-neopentyl-2-oxo-1,2,3,5-tetrahydro-4,1-thieno[2,3-e]oxazepine-3-acetic acid, (3R,5S)-7-chloro-5-(2-methoxyphenyl)-1-neopentyl-2-oxo-1,2,3,5-tetrahydro-4,1-thieno[2,3-e]oxazepine-3-acetic acid, (3R,5S)-7-chloro-1-isobutyl-5-(2-methoxyphenyl)-2-oxo-1,2,3,5-tetrahydro-4,1-thieno[2,3-e] oxazepine-3-acetic acid, (3R,5S)-7-chloro-5-(3-hydroxy-2-methoxyphenyl)-1-neopentyl-2-oxo-1,2,3,5-tetrahydro-4,1-benzothiazepine-3-acetic acid, (3R,5S)-7-chloro-5-(4-hydroxy-2-methoxyphenyl)-1-neopentyl-2-oxo-1,2,3,5-tetrahydro-4,1-benzothiazepine-3-acetic acid, (3R,5S)-7-chloro-5-(3-hydroxy-2-methoxyphenyl)-1-isobutyl-2-oxo-1,2,3,5-tetrahydro-4,1-benzothiazepine-3-acetic acid, (3R,5S)-7-chloro-5-(4-hydroxy-2-methoxyphenyl)-1-isobutyl-2-oxo-1,2,3,5-tetrahydro-4,1-benzothiazepine-3-acetic acid, (3R,5S)-7-chloro-5-(3-ethoxy-2-methoxyphenyl)-1-neopentyl-2-oxo-1,2,3,5-tetrahydro-4,1-benzothiazepine-3-acetic acid, (3R,5S)-7-chloro-5-(4-ethoxy-2-methoxyphenyl)-1-neopentyl-2-oxo-1,2,3,5-tetrahydro-4,1-benzothiazepine-3-acetic acid, (3R,5S),-7-chloro-5-(3-ethoxy-2-methoxyphenyl)-1-isobutyl-2-oxo-1,2,3,5-tetrahydro-4,1-benzothiazepine-3-acetic acid, (3R,5S)-7-chloro-5-(4-ethoxy-2-methoxyphenyl)-1-isobutyl-2-oxo-1,2,3,5-tetrahydro-4,1-benzothiazepine-3-acetic acid, (3R,5S)-7-chloro-5-(2-chloro-3-methoxyphenyl)-1-neopentyl-2-oxo-1,2,3,5-tetrahydro-4,1-benzothiazepine-3-acetic acid, (3R,5S)-7-chloro-5-(2-chloro-4-methoxyphenyl)-1-neopentyl-2-oxo-1,2,3,5-tetrahydro-4,1-benzothiazepine-3-acetic acid, (3R,5S)-7-chloro-5-(2-chloro-3-methoxyphenyl)-1-isobutyl-2-oxo-1,2,3,5-tetrahydro-4,1-benzothiazepine-3-acetic acid, (3R,5S)-7-chloro-5-(2-chloro-4-methoxyphenyl)-1-isobutyl-2-oxo-1,2,3,5-tetrahydro-4,1-benzothiazepine-3-acetic acid, (3R,5S)-7-chloro-5-(2-chloro-3-hydroxyphenyl)-1-neopentyl-2-oxo-1,2,3,5-tetrahydro-4,1-benzothiazepine-3-acetic acid, (3R,5S)-7-chloro-5-(2-chloro-4-hydroxyphenyl)-1-neopentyl-2-oxo-1,2,3,5-tetrahydro-4,1-benzothiazepine-3-acetic acid, (3R,5S)-7-chloro-5-(2-chloro-3-hydroxyphenyl)-1-isobutyl-2-oxo-1,2,3,5-tetrahydro-4,1-benzothiazepin-3-acetic acid, (3R,5S)-7-chloro-5-(2-chloro-4-hydroxyphenyl)-1-isobutyl-2-oxo-1,2,3,5-tetrahydro-4,1-benzothiazepine-3-acetic acid; and salts thereof.

The compounds represented by the formula (I) and the salts thereof [hereinafter, sometimes, abbreviated as Compound (I) including salts] are disclosed in, for example, EP-A-567026, WO95/21834 (Japanese Patent Application No. 6-15531), EP-A-645377 (Japanese Patent Application No. 6-229159), EP-A-645378 (Japanese Patent Application No. 6-229160), and it can be prepared based on the disclosure of these publications.

The compound represented by the formula (I) is preferably the compound represented by the formula (Ib):

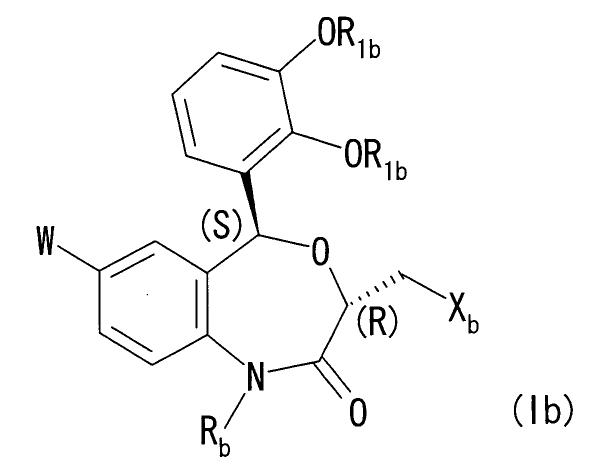

Preferable examples of the compound represented by the formula (Ib) include:
the compound in which R_{b} is a C₁₋₆ alkyl group which may have 1 to 3 substituents selected from a hydroxy group, acetyloxy, propionyloxy, t-butoxycarbonyloxy, palmitoyloxy, dimethylaminoacetyloxy and 2-aminopropionyloxy;
the compound in which R_{b} is a C₃₋₆ branched alkyl group which may have 1 to 3 substituents selected from a hydroxy group, acetyloxy, propionyloxy, t-butoxycarbonyloxy, palmitoyloxy, dimethylaminoacetyloxy and 2-aminopropionyloxy;
the compound in which R_{b} is 2,2-dimethyl-3-hydroxypropyl, 3-hydroxy-2-hydroxymethyl-2-methylpropyl, 3-acetoxy-2,2-dimethylpropyl, 3-acetoxy-2-hydroxymethyl-2-methylpropyl or 3-acetoxy-2-acetoxymethyl-2-methylpropyl;
the compound in which R_{1b} is methyl;
the compound in which W is a chlorine atom;
the compound in which X_{b} is a group represented by the formula: 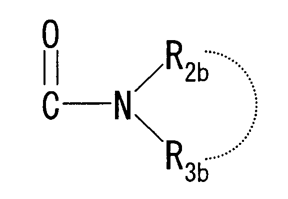 wherein, R_{2b} and R_{3b} are each a hydrogen atom, an optionally substituted hydrocarbon group, an optionally substituted heterocyclic group or an acyl group, or R_{2b} and R_{3b} may form, together with the adjacent nitrogen atom, an optionally substituted 5- or 6-membered nitrogen-containing heterocyclic ring optionally containing 1 to 3 hetero atom selected from a nitrogen atom, a sulfur atom and an oxygen atom as atoms constituting a ring;
the compound in which a group represented by X_{b}, wherein
   R_{2b} is a hydrogen atom or a C₁₋₇ alkyl group,
   R_{3b} is (1) a hydrocarbon group selected from (a) C₁₋₇ alkyl, (b) C₃₋₇ cycloalkyl, (c) C₂₋₆ alkenyl, (d) C₆₋₁₀ aryl and (e) C₆₋₁₀ aryl-C₁₋₄ alkyl [wherein, (a) C₁₋₇ alkyl, (b) C₃₋₇ cycloalkyl and (c) C₂₋₆ alkenyl may be respectively substituted with 1 to 4 substituent selected from (i) carboxyl group optionally esterified with C₁₋₆ alkyl or C₆₋₁₀ aryl-C₁₋₄ alkyl, (ii) phosphate group optionally mono- or di-substituted with C₁₋₆ alkyl or C₂₋₇ alkanoyloxy-C₁₋₆ alkyl, (iii) a sulfonate group, (iv) sulfonamide group optionally substituted with C₁₋₆ alkyl or C₆₋₁₀ aryl-C₁₋₄ alkyl, (v) hydroxy group optionally alkylated with C₁₋₃ alkyl, (vi) sulfhydryl group optionally alkylated with C₁₋₃ alkyl, (vii) a carbamoyl group, (viii) phenyl group optionally substituted with 1 to 5 substituents selected from a hydroxy group, a chlorine atom, a fluorine atom, aminosulfonyl and amino group optionally mono- or di-substituted with C₁₋₃ alkyl, (ix) amino group optionally mono- or di-substituted with C₁₋₃ alkyl, (x) cyclic amino group derived from piperidine, pyrrolidine, morpholine, thiomorpholine, piperazine, 4-methylpiperazine, 4-benzylpiperazine, 4-phenylpiperazine, 1,2,3,4-tetrahydroisoquinoline or phthalimide, optionally substituted with C₁₋₃ alkyl, benzyl or phenyl and (xi) a 5-to 6-membered aromatic heterocyclic group derived from pyridine, imidazole, indole or tetrazole; and (d) C₆₋₁₀ aryl and (e) C₆₋₁₀ aryl-C₁₋₄ alkyl may be respectively substituted with 1 to 4 substituent selected from (i) carboxyl group optionally esterified with C₁₋₄ alkyl, (ii) phosphate group optionally mono- or di-substituted with C₁₋₆ alkyl or C₂₋₇ alkanoyloxy-C₁₋₆ alkyl, (iii) a sulfonate group, (iv) a C₁₋₄ alkylsulfonyl, C₆₋₁₀ arylsulfonyl or C₆₋₁₀ aryl-C₁₋₄ alkylsulfonyl group, (v) sulfonamide group optionally substituted with C₁₋₆ alkyl or C₆₋₁₀ aryl-C₁₋₄ alkyl, (vi) C₁₋₃ alkyl group optionally substituted with carboxyl group optionally esterified with C₁₋₄ alkyl, phosphate group optionally mono- or di-substituted with C₁₋₆ alkyl, a sulfonate group, C₁₋₄ alkylsulfonyl, C₆₋₁₀ arylsulfonyl or C₆₋₁₀ aryl-C₁₋₄ alkylsulfonyl, sulfonamide group optionally substituted with C₁₋₆ alkyl or C₆₋₁₀ aryl-C₁₋₄ alkyl and (vii) a halogen atom],
   (2) tetrazolyl, 4,5-dihydro-5-oxo-1,2,4-oxadiazolyl, 4,5-dihydro-5-thioxo-1,2,4-oxadiazolyl, 2,3-dihydro-3-oxo-1,2,4-oxadiazolyl, 2,3-dihydro-3-thioxo-1,2,4-oxadiazolyl, 3,5-dioxo-1,2,4-oxadiazolydinyl, 4,5-dihydro-5-oxo-isoxazolyl, 4,5-dihydro-5-thioxo-isoxazolyl, 2,3-dihydro-2-oxo-1,3,4-oxadiazolyl, 2,3-dihydro-3-oxo-1,2,4-tetrazolyl or 2,3-dihydro-3-thioxo-1,2,4-tetrazolyl group, or
   (3) an acyl group selected from (i) a C₂₋₇ alkanoyl group which may be optionally substituted with 1 to 2 halogen atoms, and (ii) a C₆₋₁₀ arylsulfonyl group optionally substituted with 1 to 4 substituents selected from C₁₋₃ alkyl, C₁₋₃ alkoxy and a halogen atom, a C₁₋₄ alkylsulfonyl group or a C₆₋₁₀ aryl-C₁₋₄ alkylsulfonyl group, or R_{2b} and R_{3b} may be taken together with the adjacent nitrogen atom to form 5- to 6-membered ring derived from piperidine, piperazine, pyrrolidine, 2-oxopiperazine, 2,6-dioxopiperazine, morpholine or thiomorpholine [wherein, the 5-membered or 6-membered ring is optionally substituted with 1 to 4 substituent selected from (A) hydroxy group optionally substituted with C₁₋₃ alkyl or C₂₋₇ alkanoyl, (B) carboxyl group optionally esterified with C₁₋₆ alkyl or C₆₋₁₀ aryl-C₁₋₄ alkyl, (C) phosphate group optionally mono- or di-substituted with C₁₋₆ alkyl or C₂₋₇ alkanoyloxy-C₁₋₆ alky, (D) a sulfonate group, (E) sulfonamide group optionally substituted with C₁₋₆ alkyl or C₆₋₁₀ aryl-C₁₋₄ alkyl, (F) C₁₋₆ alkyl and C₂₋₅ alkenyl, each of which is optionally substituted with a carboxyl group optionally esterified with C₁₋₆ alkyl or C₆₋₁₀ aryl-C₁₋₄ alkyl; a phosphate group optionally mono- or di-substituted with C₁₋₆ alkyl or C₂₋₇ alkanoyloxy-C₁₋₆ alkyl; a sulfonate group; sulfonamide group optionally substituted with C₁₋₆ alkyl or C₆₋₁₀ aryl-C₁₋₄ alkyl; a hydroxy group optionally substituted with C₁₋₃ alkyl or C₂₋₇ alkanoyl; a sulfhydryl group optionally alkylated with C₁₋₃ alkyl; a carbamoyl group; phenyl optionally substituted with 1 to 5 substituents selected from a hydroxy group, a halogen atom, an aminosulfonyl and an amino group optionally substituted with C₁₋₃ alkyl; an amino group optionally mono- or di-substituted with C₁₋₃ alkyl; or tetrazolyl, (G) amino group optionally mono- or di-substituted with C₁₋₃ alkyl, (H) a cyclic amino group derived from piperidine, pyrrolidine, morpholine, thiomorpholine, 4-methylpiperazine, 4-benzylpiperazine or 4-phenylpiperazine, (I) a cyano group, (J) a carbamoyl group, (K) an oxo group, (L) a tetrazolyl or 2,5-dihydro-5-oxo-1,2,4-oxadiazolyl group, (M) carbamoyl group optionally substituted with C₆₋₁₀ arylsulfonyl, C₁₋₄ alkylsulfonyl or C₆₋₁₀ aryl-C₁₋₄ alkylsulfonyl, (N) sulfhydryl group optionally alkylated with C₁₋₃ alkyl, and (O) phenyl group which may be substituted with 1 to 5 substituents selected from a hydroxy group, a halogen atom, aminosulfonyl and amino group optionally substituted with C₁₋₃ alkyl];
the compound wherein in a group represented by X_{b} , R_{2b} and R_{3b} may be taken together with the adjacent nitrogen atom of a carbamoyl group to form a 5- or 6-membered ring derived from piperidine, piperazine, pyrrolidine, 2-oxopiperazine or 2,6-dioxopiperazine, and the 5- or 6-membered ring is optionally substituted with C₁₋₆ alkyl group which may have 1 to 2 substituents selected from (i) carboxyl group optionally esterified with C₁₋₆ alkyl or C₆₋₁₀ aryl-C₁₋₄ alkyl, (ii) phosphate group optionally mono- or di-substituted with C₁₋₆ alkyl or C₂₋₇ alkanoyl-C₁₋₆ alkyl, (iii) a sulfonate group, (iv) sulfonamide group optionally substituted with C₁₋₆ alkyl or C₆₋₁₀ aryl-C₁₋₄ alkyl, (v) hydroxy group optionally alkylated with C₁₋₃, (vi) sulfhydryl group optionally alkylated with C₁₋₃ alkyl, (vii) a carbamoyl group, (viii) phenyl group which may be substituted with 1 to 5 substituents selected from a hydroxy group, a halogen atom, aminosulfonyl and amino optionally substituted with C₁₋₃ alkyl, (ix) amino group optionally mono- or di-substituted with C₁₋₃ alkyl, and (x) a tetrazolyl group;
the compound wherein in a group represented by X_{b}, R_{2b} is a hydrogen atom or C₁₋₇ alkyl and R_{3b} is C₁₋₄ alkylsulfonyl; the compound in which a heterocyclic group represented by X_{b} is tetrazolyl, 4,5-dihydro-5-oxo-1,2,4-oxadiazolyl, 4,5-dihydro-5-thioxo-1,2,4-oxadiazolyl, 2,3-dihydro-3-oxo-1,2,4-oxadiazolyl, 2,3-dihydro-3-thioxo-1,2,4-oxadiazolyl, 3,5-dioxo-1,2,4-oxadiazolydinyl, 4,5-dihydro-5-oxo-isoxazolyl, 4,5-dihydro-5-thioxo-isoxazolyl, 2,3-dihydro-2-oxo-1,3,4-oxadiazolyl, 2,3-dihydro-3-oxo-1,2,4-tetrazolyl or 2,3-dihydro-3-thioxo-1,2,4-tetrazolyl;
the compound in which R_{1b} is methyl, W is a chlorine atom, R_{b} is C₃₋₆ branched alkyl which is substituted with 1 to 3 substituents selected from a hydroxy group, acetyloxy, propionyloxy, tert-butoxycarbonyloxy, palmitoyloxy, dimethylaminoacetyloxy and 2-aminopropionyloxy, and X_{b} is a group represented by the formula: 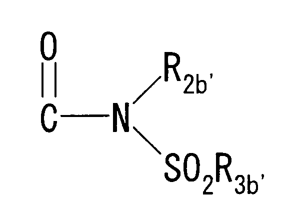 wherein, R_{2b'} is a hydrogen atom or C₁₋₇ alkyl and R_{3b'} is C₁₋₄ alkyl;
the compound in which R_{1b} is methyl, W is a chlorine atom, R_{b} is C₃₋₆ branched alkyl which is substituted with 1 to 3 substituents selected from a hydroxy group, acetyloxy, propionyloxy, tert-butoxycarbonyloxy, palmitoyloxy, dimethylaminoacetyloxy and 2-aminopropionyloxy, and X_{b} is a group represented by the formula: 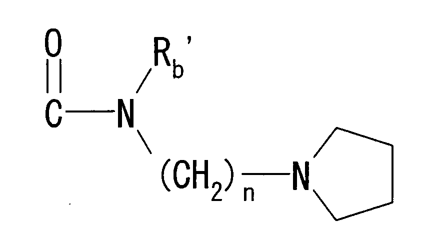 wherein, R_{b'} is a hydrogen atom or C₁₋₇ alkyl, and n is an integer of 1 to 5;
the compound in which R_{1b} is methyl, W is a chlorine atom, R_{b} is C₃₋₆ branched alkyl which is substituted with 1 to 3 substituents selected from a hydroxy group, acetyloxy, propionyloxy, tert-butoxycarbonyloxy, palmitoyloxy, dimethylaminoacetyloxy and 2-aminopropionyloxy, and X_{b} is a group represented by the formula: 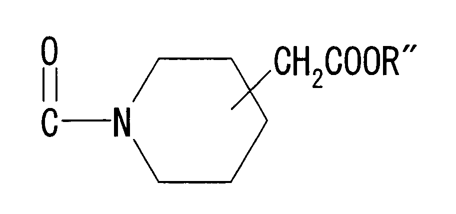 wherein, R" is a hydrogen atom or C₁₋₄ alkyl;
the compound in which R_{1b} is methyl, W is a chlorine atom, R_{b} is C₃₋₆ branched alkyl which is substituted with 1 to 3 substituents selected from a hydroxy group, acetyloxy, propionyloxy, tert-butoxycarbonyloxy, palmitoyloxy, dimethylaminoacetyloxy and 2-aminopropionyloxy, and X_{b} is tetrazolyl;
the compound in which R_{b} is lower alkyl optionally substituted with 1 or 2 hydroxy groups, and X_{b} is (1) carbamoyl group optionally substituted with a hydrocarbon group selected from (a) C₁₋₇ alkyl, (b) C₃₋₇ cycloalkyl, (c) C₂₋₆ alkenyl, (d) C₆₋₁₀ aryl and (e) C₇₋₁₄ arylalkyl [wherein, (a) C₁₋₇ alkyl, (b) C₃₋₇ cycloalkyl, and (c) C₂₋₆ alkenyl may be respectively substituted with 1 to 4 substituent selected from (i) carboxyl group optionally esterified with C₁₋₆ alkyl or C₇₋₁₀ arylalkyl, (ii) a phosphate group, (iii) a sulfonate group, (iv) sulfonamide group optionally substituted with C₁₋₆ alkyl or C₇₋₁₀ arylalkyl, (v) hydroxy group optionally alkylated with C₁₋₃ alkyl, (vi) sulfhydryl group optionally alkylated with C₁₋₃ alkyl, (vii) a carbamoyl group, (viii) an phenyl group optionally substituted with substituents selected from a hydroxy group, a chlorine atom, a fluorine atom, aminosulfonyl and amino group optionally mono- or di-substituted with C₁₋₃ alkyl, (ix) amino group optionally mono- or di-substituted with C₁₋₃ alkyl, and (x) cyclic amino group derived from piperidine, pyrrolidine, morpholine, thiomorpholine, piperazine, 4-methylpiperazine, 4-benzylpiperazine, or 4-phenylpiperazine, which may be substituted with C₁₋₃ alkyl, benzyl or phenyl and (xi) 5- or 6-memebered aromatic heterocyclic group derived from pyridine, imidazole, indole or tetrazole, and (d) C₆₋₁₀ aryl and (e) C₇₋₁₄ arylalkyl may be respectively substituted with 1 to 4 substituent selected from (i) carboxyl group optionally esterified with C₁₋₄ alkyl, (ii) a phosphate group, (iii) a sulfonate group, (iv) sulfonamide group optionally substituted with C₁₋₆ alkyl or C₇₋₁₀ arylalkyl, (v) C₁₋₃ alkyl group optionally substituted with carboxyl group optionally esterified with C₁₋₄ alkyl, a phosphate group, a sulfonate group, or sulfonamide group optionally substituted with C₁₋₆ alkyl or C₇₋₁₀ arylalkyl, and (iv) a halogen atom],
   (2) tetrazolyl, 4,5-dihydro-5-oxo-1,2,4-oxadiazolyl, 4,5-dihydro-5-thioxo-1,2,4-oxadiazolyl, 2,3-dihydro-3-oxo-1,2,4-oxadiazolyl, 2,3-dihydro-3-thioxo-1,2,4-oxadiazolyl, 3,5-dioxo-1,2,4-oxadiazolydinyl, 4,5-dihydro-5-oxo-isoxazolyl, 4,5-dihydro-5-thioxo-isoxazolyl, 2,3-dihydro-2-oxo-1,3,4-oxadiazolyl, 2,3-dihydro-3-oxo-1,2,4-tetrazolyl or 2,3-dihydro-3-thioxo-1,2,4-tetrazolyl group,
   (3) carbamoyl group optionally substituted with an acyl group selected from (i) a C₂₋₇ alkanoyl group which is optionally substituted with 1 to 2 halogen atoms, and (ii) a C₆₋₁₀ arylsulfonyl group, a C₁₋₄ alkylsulfonyl group or a C₇₋₁₄ arylalkylsulfonyl group, each of which may be optionally substituted with 1 to 4 substituents selected from C₁₋₃ alkyl, C₁₋₃ alkoxy and a halogen atom, or
   (4) a cyclic amino carbonyl group derived from piperidine, piperazine, pyrrolidine, 2-oxopiperazine, 2,6-dioxopiperazine, morpholine and thiomorpholine [wherein, the cyclic amino carbonyl group is optionally substituted with 1 to 4 substituent selected from (A) a hydroxy group, (B) carboxyl group optionally esterified with C₁₋₄ alkyl, (C) a phosphate group, (D) a sulfonate group, (E) sulfonamide group optionally substituted with C₁₋₆ alkyl or C₇₋₁₀ arylalkyl, (F) C₁₋₃ alkyl or C₂₋₅ alkenyl, each of which may be substituted with the above-mentioned (A), (B), (C), (D) or (E), (G) amino group optionally mono- or di-substituted with C₁₋₃ alkyl, (H) a cyclic amino group derived from piperidine, pyrrolidine, morpholine, thiomorpholine, 4-methylpiperazine, 4-benzylpiperazine or 4-phenylpiperazine, (I) a cyano group, (J) a carbamoyl group, (K) oxo, (L) C₁₋₃ alkoxy, (M) a heterocyclic group derived from tetrazolyl or 2,5-dihydro-5-oxo-1,2,4-oxadiazolyl, and (N) carbamoyl group optionally substituted with C₆₋₁₀ arylsulfonyl, C₁₋₄ alkylsulfonyl or C₇₋₁₄ arylalkylsulfonyl];
the compound in which R_{b} is a 2,2-dimethyl-3-hydroxypropyl group; or the like.

In the aforementioned formula, the lower alkyl group represented by R_{b} includes, for example, C₁₋₆ alkyl such as methyl, ethyl, n-propyl, isopropyl, n-butyl, isobutyl, n-pentyl, isopentyl, neopentyl and hexyl. Among these, a C₃₋₆ alkyl group is preferable, and a C₄₋₅ alkyl group is more preferable. In particular, a C₄₋₅ branched alkyl group such as isobutyl, neopentyl or the like, is preferable.

The substituent of the lower alkyl represented by R_{b} includes, for example, hydroxy group optionally substituted with C₂₋₂₀ alkanoyl or C₁₋₇ alkyl or the like. Examples of these substituents include a hydroxy group, acetyloxy, propionyloxy, tert-butoxycarbonyloxy, palmitoyloxy, dimethylaminoacetyloxy and 2-aminopropionyloxy.

One to three of these substituents may be present at their substitutable positions.

In addition, examples of the optionally substituted lower alkyl represented by R_{b} include 2,2-dimethyl-3-hydroxypropyl, 3-hydroxy-2-hydroxymethyl-2-methylpropyl, 3-acetoxy-2,2-dimethylpropyl, 3-acetoxy-2-hydroxymethyl-2-methyl-propyl and 3-acetoxy-2-acetoxymethyl-2-methylpropyl.

The optionally substituted carbamoyl group represented by X_{b} includes the group represented by the formula:

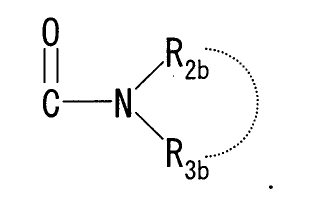

Examples of the "optionally substituted hydrocarbon group" represented by R_{2b} and R_{3b} include an optionally substituted C₁₋₇ linear or branched alkyl group (e.g., methyl, ethyl, n-propyl, isopropyl, n-butyl, isobutyl, 1,1-dimethylethyl, n-pentyl, 3-methylbutyl, 2-methylbutyl, 1-methylbutyl, 1-ethylpropyl, n-hexyl, 4-methylpentyl, 3-methylpentyl, 2-methylpentyl, 2-ethylbutyl, 1-ethylbutyl, neopentyl, hexyl, heptyl), an optionally substituted C₃₋₇ cycloalkyl group (cyclopropyl, cyclobutyl, cyclopentyl, cyclohexyl, cyclohexylmethyl, etc.), an optionally substituted C₂₋₆ linear or branched alkenyl group (e.g., vinyl, allyl, isopropenyl, 2-methylallyl, 1-propenyl, 2-methyl-1-propenyl, 2-methyl-2-propenyl, 1-butenyl, 2-butenyl, 3-butenyl, 2-ethyl-1-butenyl, 2-methyl-2-butenyl, 3-methyl-2-butenyl, 1-pentenyl, 2-pentenyl, 3-pentenyl, 4-pentenyl, 4-methyl-3-pentenyl, 1-hexenyl, 2-hexenyl, 3-hexenyl, 4-hexenyl, 5-hexenyl etc.), an optionally substituted C₆₋₁₀ aryl group (e.g., phenyl and naphthyl group) and an optionally substituted C₇₋₁₄ arylalkyl group (e.g., benzyl, phenethyl and naphthylmethyl).

The substituent of the "optionally substituted C₁₋₇ linear or branched alkyl group, optionally substituted C₃₋₇ cycloalkyl group and optionally substituted C₂₋₆ linear or branched alkenyl group" includes, for example, carboxyl group optionally esterified with C₁₋₆ alkyl group or C₆₋₁₀ aryl-C₁₋₄ alkyl group (e.g., methyl, ethyl, propyl, isopropyl, butyl, tert-butyl, phenyl, benzyl, etc.), phosphate group optionally mono- or di-substituted with C₁₋₆ alkyl group (e.g., methyl, ethyl, n-propyl, isopropyl, n-butyl, isobutyl, n-pentyl, isopentyl, neopentyl, hexyl, etc.) or C₂₋₇ alkanoyloxy-C₁₋₆ alkyl such as acetyloxymethyl or pivaloyloxymethyl group, a sulfonate group, sulfonamide group optionally substituted which is substituted with C₁₋₆ alkyl group or C₆₋₁₀ aryl-C₁₋₄ alkyl group (e.g., methyl, ethyl, propyl, isopropyl, butyl, tert-butyl, phenyl, benzyl, etc.), hydroxy group and sulfhydryl group, each optionally alkylated with C₁₋₃ alkyl group (e.g., methyl, ethyl, propyl, etc.), a carbamoyl group, phenyl group optionally substituted with 1 to 5 substituents [e.g., a hydroxy group, chlorine, fluorine, an aminosulfonyl group or amino group optionally substituted with C₁₋₃ alkyl group (e.g., methyl, ethyl, propyl, etc.)], an amino group optionally mono- or di-substituted with C₁₋₃ alkyl group (e.g., methyl, ethyl, propyl, etc.), a cyclic amino group (e.g., 5- or 6-membered cyclic amino group derived from cyclic amine such as piperidine, pyrrolidine, morpholine, thiomorpholine, piperazine, 4-methylpiperazine, 4-benzylpiperazine, 4-phenylpiperazine, 1,2,3,4-tetrahydroisoquinoline, phthalimide, or the like, which may be optionally substituted with a C₁₋₃ alkyl group, benzyl, phenyl or the like, and optionally contains an oxygen atom or a sulfur atom as additional atoms constituting a ring) and 5- to 6-membered aromatic heterocyclic ring containing 1 to 4 hetero atoms selected from N, O, S (e.g. pyridine, imidazole, indole, tetrazole, etc).

In addition, as for the substituent of C₆₋₁₀ aryl group and C₆₋₁₀ aryl-C₁₋₄ alkyl group as the substituent of an optionally substituted amino group which forms a carbamoyl group of the "optionally substituted carbamoyl group" represented by X_{b} includes an optionally esterified carboxyl group with C₁₋₄ alkyl group (methyl, ethyl, propyl, tert-butyl group, etc.), , phosphate group optionally mono- or di-substituted with C₁₋₆ alkyl group (methyl, ethyl, n-propyl, isopropyl, n-butyl, isobutyl, n-pentyl, isopentyl, neopentyl, hexyl) or C₂₋₇ alkanoyloxy-C₁₋₆ alkyl such as pivaloyloxymethyl group or acetyloxymethyl group, a sulfonate group, C₁₋₄ alkylsulfonyl, C₆₋₁₀ arylsulfonyl or C₆₋₁₀ aryl-C₁₋₄ alkylsulfonyl, sulfonamide group optionally substituted with a C₁₋₆ alkyl group or a C₆₋₁₀ aryl-C₁₋₄ alkyl group (e.g., methyl, ethyl, propyl, isopropyl, butyl, tert-butyl, benzyl, etc.) and an optionally esterified carboxyl group with a C₁₋₄ alkyl group, phosphate group optionally mono- or di-substituted with a C₁₋₆ alkyl group such as methyl, ethyl, n-propyl, isopropyl, n-butyl, isobutyl, n-pentyl, isopentyl, neopentyl, hexyl, or the like or C₂₋₇ alkanoyloxy-C₁₋₆ alkyl such as a pivaloyloxymethyl group, , C₁₋₃ alkyl group (e.g., methyl, ethyl, propyl and isopropyl) optionally substituted with a sulfonate group and sulfonamide group optionally substituted with C₁₋₆ alkyl or a C₆₋₁₀ aryl-C₁₋₄ alkyl group, and a halogen atom (fluorine and chlorine), and the like.

The "hydrocarbon group" may have 1 to 5 substituents at a substitutable position.

The "optionally substituted heterocyclic group" represented by R_{2b} and R_{3b} may have 1 to 2 substituent preferably such as an oxo group or a thioxo group, and may be preferably a heterocyclic group having a hydrogen which can be deprotonated. These heterocyclic groups is preferably a 5- to 6-membered heterocyclic group containing 1 to 4, preferably 2 to 3 hetero atoms selected from S, O or N. Specific examples include tetrazolyl, 4,5-dihydro-5-oxo-1,2,4-oxadiazolyl, 4,5-dihydro-5-thioxo-1,2,4-oxadiazolyl, 2,3-dihydro-3-oxo-1,2,4-oxadiazolyl, 2,3-dihydro-3-thioxo-1,2,4-oxadiazolyl, 3,5-dioxo-1,2,4-oxadiazolydinyl, 4,5-dihydro-5-oxo-isoxazolyl, 4,5-dihydro-5-thioxo-isoxazolyl, 2,3-dihydro-2-oxo-1,3,4-oxadiazolyl, 2,3-dihydro-3-oxo-1,2,4-tetrazolyl and 2,3-dihydro-3-thioxo-1,2,4-tetrazolyl. Among these, tetrazolyl group is preferable.

Examples of the "acyl group" represented by R_{2b} and R_{3b} include an acyl carboxylate group derived from carboxylate (e.g., C₂₋₇ acyl carboxylate group such as acetyl, propionyl, butyryl, benzoyl, etc.) and C₆₋₁₀ arylsulfonyl group, a C₁₋₄ alkylsulfonyl group and C₆₋₁₀ aryl-C₁₋₄ alkylsulfonyl group which may optionally have a substituent (e.g., methylsulfonyl, ethylsulfonyl, phenylsulfonyl, naphthylsulfonyl, phenylmethylsulfonyl, phenylethylsulfonyl, naphthylmethylsulfonyl, naphthylethylsulfonyl, etc.). The substituents of aryl, alkyl and arylalkylsulfonyl group include C₁ to C₃ alkyl (e.g., methyl, ethyl, propyl, etc.), C₁₋₃ alkoxy (e.g., methoxy, ethoxy, propoxy, etc.), a halogen atom (e.g., chlorine, fluorine, bromine), and 1 to 4, preferably 1 to 2, thereof may be present at a substitutable position.

The aforementioned acyl carboxylate group may have 1 to 2 halogen atoms (chlorine, fluorine, bromine) as a substituent.

Examples of the cyclic amino group optionally substituted with C₁₋₃ alkyl, C₂₋₇ alkanoyl or the like, which is formed by taking together R_{2b} and R_{3b} with the adjacent nitrogen atom of carbamoyl group, include a group derived from 5- to 6-membered cyclic amine such as cyclic amine (e.g. piperazine, piperidine, pyrrolidine, piperazin-2-one, piperazine-2,6-dione, morpholine, thiomorpholine, etc) which may additionally contain 1 to 3 hetero atoms selected from a nitrogen atom, a sulfur atom, or an oxygen atom as atoms constituting a ring. Such cyclic amino group may have 1 to 4, preferably 1 to 2 substituents. Examples of the substituents include hydroxy group optionally substituted with C₁₋₃ alkyl group or C₂₋₇ alkanoyl, carboxyl group optionally esterified with C1-4 alkyl group (methyl, ethyl, propyl, tert-butyl, etc.) and C₇₋₁₀ arylalkyl, phosphate group optionally mono- or di-substituted with C₁₋₆ alkyl or C₂₋₇ alkanoyloxy-C₁₋₆ alkyl group (acetyloxymethyl group, pivaloyloxymethyl group), a sulfonate group, and sulfonamide group optionally substituted with C₁₋₆ alkyl or C₆₋₁₀ aryl-C₁₋₄ alkyl group (e.g., methyl, ethyl, propyl, isopropyl, butyl, tert-butyl, benzyl, etc.), C₁₋₆ alkyl and C₂₋₅ alkenyl, each optionally substituted with "carboxyl group optionally esterified with C₁₋₆ alkyl or C₆₋₁₀ aryl-C₁₋₄ alkyl, phosphate group optionally mono- or di-substituted with C₁₋₆ alkyl or C₂₋₇ alkanoyloxy-C₁₋₆ alkyl group (acetyloxymethyl group, pivaloyloxymethyl group, etc.), a sulfonate group, sulfonamide group optionally substituted with C₁₋₆ alkyl or C₆₋₁₀ aryl-C₁₋₄ alkyl group, hydroxy group optionally substituted with C₁₋₃ alkyl or C₂₋₇ alkanoyl, sulfhydryl group optionally alkylated with C₁₋₃ alkyl, a carbamoyl group, phenyl group optionally substituted with 1 to 5 substituents (e.g., a hydroxy group, a halogen atom, aminosulfonyl, amino group optionally substituted with C₁₋₃ alkyl, etc.), amino group optionally mono- or di-substituted with C₁₋₃ alkyl or tetrazolyl group", amino group optionally mono- or di-substituted with C₁₋₃ alkyl (e.g., methyl, ethyl, propyl, etc.), a cyclic amino group (a group derived from 5- to 6-membered cyclic amine which may contain additional hetero atoms as atoms constituting a ring selected from a nitrogen atom, a sulfur atom, or an oxygen and which may be substituted with C₁-C₃ alkyl, benzyl, phenyl, or the like, for example, piperidine, pyrrolidine, morpholine, thiomorpholine, 4-methylpiperazine, 4-benzylpiperazine, 4-phenylpiperazine, or the like), a cyano group, a carbamoyl group, an oxo group, C₁₋₃ alkoxy (e.g., methoxy, ethoxy, ethylenedioxy, etc.), heterocyclic group optionally substituted with an oxo group or thioxo group having a hydrogen which can be deprotonated as mentioned above (e.g. tetrazolyl, 2,5-dihydro-5-oxo-1,2,4-oxadiazolyl, etc.), C₆₋₁₀ arylsulfonyl, C₆₋₁₀ aryl-C₁₋₄ alkylsulfonyl and C₁₋₄ alkylsulfonyl (methylsulfonyl, ethylsulfonyl, propyl sulfonyl, butylsulfonyl, isopropyl sulfonyl, tert-butylsulfonyl, phenyl, sulfonyl, benzylsulfonyl, etc.) as exemplified for the substituent of an optionally substituted amino group which forms carbamoyl of the "optionally substituted carbamoyl group" represented by X, sulfhydryl group optionally alkylated with C₁₋₃ alkyl, or carbamoyl group substituted with phenyl optionally substituted with 1 to 5 substituents (e.g. a hydroxy group, a halogen atom, an aminosulfonyl and amino group optionally substituted with C₁₋₃ alkyl) .

Examples of the carbamoyl group, which may have substituents represented by X_{b} include:

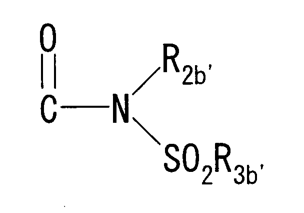

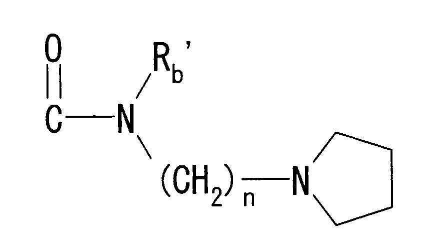

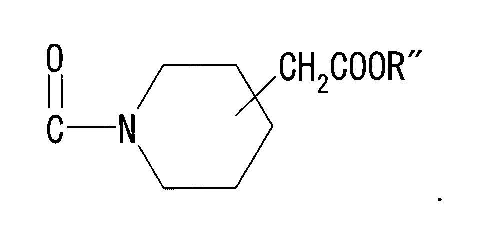

R_{2b'} and R_{b'} include a hydrogen atom and C₁₋₇ alkyl. The hydrogen atom is particularly preferable.

The C₁₋₇ alkyl represented by R_{2b}, R_{2b'} and R_{b'} includes those such as the aforementioned C₁₋₇ alkyl of the "hydrocarbon group".

R" includes a hydrogen atom and C₁₋₄ alkyl. The hydrogen atom is particularly preferable.

C₁₋₄ alkyl represented by R_{3b}, and R" includes, for example, methyl, ethyl, propyl, isopropyl, n-butyl, tert-butyl.

As for the optionally substituted heterocyclic group having a hydrogen which can be deprotonated represented by X_{b}, a nitrogen-containing (preferably including 1 to 4 nitrogen atoms) 5- to 6-membered heterocyclic ring having active proton of Broensted acid is preferable, and those containing 1 to 4, preferably 2 to 3 of a nitrogen atom, a sulfur atom, an oxygen atom may be preferable. These substituents include, an oxo group and a thioxo group, and 1 to 2 (preferably 1) of such substituents may be present. The "optionally substituted heterocyclic group having a hydrogen which can be deprotonated" represented by X includes, for example, a group as exemplified as the "optionally substituted heterocyclic group" as the substituent of the "optionally substituted carbamoyl group" represented by X such as tetrazolyl, 2,5-dihydro-5-oxo-1,2,4-oxadiazolyl and the like.

Examples of the "lower alkyl group" represented by R_{1b} include a C₁-C₆ alkyl group such as methyl, ethyl, n-propyl, isopropyl, n-butyl, tert-butyl, pentyl, hexyl and the like. Among these, C₁-C₃ alkyl group is preferable. In the view of pharmacological activity, a methyl group is particularly preferable as R_{1b}.

Examples of the "halogen atom" represented by W include chlorine, fluorine, bromine, iodine atoms. The chlorine atom is particularly preferable.

Examples of the salts of the compound represented by the formula (Ib), include pharmacologically acceptable salts, for example, inorganic salts such as hydrochloride, hydrobromate, sulfate, nitrate, phosphate and the like; organic salts such as acetate, tartrate, citrate, fumarate, maleate, toluenesulfonate, methanesulfonate and the like; metal salts such as a sodium salt, a potassium salt, a calcium salt, an aluminum salt and the like; and basic salts such as a triethylamine salt, a guanidine salt, an ammonium salt, a hydrazine salt, a quinine salt, a cinchonine salt and the like.

In addition, hydrates as well as non-hydrates of the compound represented by the formula (Ib) are included within the scope of the present invention.

The compound represented by the formula (Ib) and salts thereof contains asymmetric carbon atoms at 3- and 5-positions, herein the trans isomer wherein the substituents on 3- and 5-positions are directed in the opposite direction relative to the surface of a 7-membered ring is preferable, and the isomer wherein the absolute configuration at 3-position is R-configuration, and the absolute configuration at 5-position is S-configuration is particularly preferable.

Among the compounds represented by the formula (Ib) and salts thereof, the following concrete compounds are preferable.

N-methanesulfonyl-[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetamide, N-methanesulfonyl-[(3R,5 S)-7-chloro-5-(2,3-dimethoxyphenyl)-1-(3-hydroxy-2-hydroxymethyl-2-methylpropyl)-2-oxo-1,2,3,5-tetrahydro-4,1-benzoxazepin-3-yl]acetamide, N-[2-(pyrrolidin-1-yl)ethyl]-[(3R,5S)-7-chloro-5-(2,3-dimethoxyphenyl)-1-(3-hydroxy-2-hydroxymethyl-2-methylpropyl)-2-oxo-1,2,3,5-tetrahydro-4,1-benzoxazepin-3-yl]acetamide, N-[2-(pyrrolidin-1-yl)ethyl]-[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetamide, N-methanesulfonyl-[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetamide, N-methanesulfonyl-[(3R,5S)-1-(3-acetoxy-2-acetoxymethyl-2-methylpropyl)-7-chloro-5-(2,3-dimethoxyphenyl)-2-oxo-1,2,3,5-tetrahydro-4,1-benzoxazepin-3-yl]acetamide, N-[[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetyl]piperidin-4-acetic acid, N-[[(3R,5S)-1-(3-hydroxy-2,2-dimethylpropyl)-7-chloro-5-(2,3-dimethoxyphenyl)-2-oxo-1,2,3,5-tetrahydro-4,1-benzoxazepin-3-yl]acetyl]piperidin-4-acetic acid, N-[[(3R,5S)-1-(2,2-dimethylpropyl)-7-chloro-5-(2,3-dimethoxyphenyl)-2-oxo-1,2,3,5-tetrahydro-4,1-benzoxazepin-3-yl]acetyl]piperidin-4-acetic acid, N-[[(3R,5S)-1-(3-acetoxy-2-acetoxymethyl-2-methylpropyl)-7-chloro-5-(2,3-dimethoxyphenyl)-2-oxo-1,2,3,5-tetrahydro-4,1-benzoxazepin-3-yl]acetyl]piperidin-4-acetic acid, ethyl N-[[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetyl]piperidine-4-acetate, ethyl N-[[(3R,5S)-1-(3-acetoxy-2-acetoxymethyl-2-methylpropyl)-7-chloro-5-(2,3-dimethoxyphenyl)-2-oxo-1,2,3,5-tetrahydro-4,1-benzoxazepin-3-yl]acetyl]piperidine-4-acetate, (3R,5S)-7-chloro-5-(2,3-dimethoxyphenyl)-1-(3-hydroxy-2,2-dimethylpropyl)-1,2,3,5-tetrahydro-3-[1H (or 3H)-tetrazol-5-yl]methyl-4,1-benzoxapin-2-one, (3R,5S)-7-chloro-5-(2,3-dimethoxyphenyl)-1-(3-hydroxy-2-hydroxymethyl-2-methylpropyl)-1,2,3,5-tetrahydro-3-[1H (or 3H)-tetrazol-5-yl]methyl-4,1-benzoxazepin-2-one, (3R,5S)-1-(3-acetoxy-2,2-dimethylpropyl)-7-chloro-5-(2,3-dimethoxyphenyl)-1,2,3,5-tetrahydro-3-[1H (or 3H)-tetrazol-5-yl]methyl-9,1-benzoxazepin-2-one, (3R,5S)-1-(3-acetoxy-2-acetoxymethyl-2-methylpropyl)-7-chloro-5-(2,3-dimethoxyphenyl)-1,2,3,5-tetrahydro-3-[1H (or 3H)-tetrazol-5-yl]methyl-4,1-benzoxazepin-2-one, N-[2-(pyrrolidin-1-yl)ethyl]-[(3R,5S)-7-chloro-5-(2,3-dimethoxyphenyl)-1-neopentyl-2-oxo-1,2,3,5-tetrahydro-4,1-benzoxazepin-3-yl]acetamide, and the like.

The compound represented by the formula (Ib) and salts thereof can be prepared by the methods disclosed in the publications, for example, EP-A-567026, WO95/21834 (PCT application based on Japanese Patent Application No. 6-15531), EP-A-645377 (an application based on Japanese Patent Application No. 6-229159), EP-A-645378 (an application based on Japanese Patent Application No. 6-229160), WO97/10224 and the like, or the methods similar thereto.

The compound represented by the formula (Ic) is preferable as a compound represented by the formula (I).

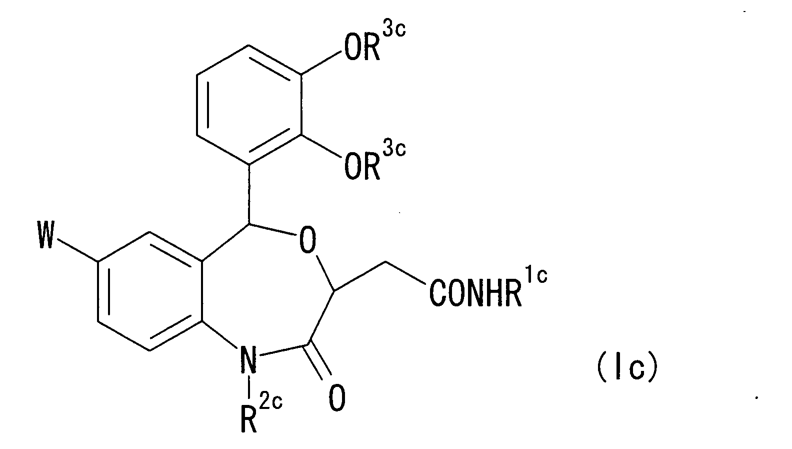

Preferable examples of the compound represented by the formula (Ic) include:
the compound in which R^{1c} is a 3-carboxypropyl group, a 1-carboxyethyl group, or a C₃₋₆ linear alkyl-sulfonyl group, a (carboxy-C₅₋₇ cycloalkyl)-C₁₋₃ alkyl group, a (carboxyfuryl)-alkyl group, a carboxy-C₆₋₁₀ aryl group, a (carboxy-C₂₋₃ alkyl) -C₆₋₁₀ aryl group or a (carboxy-C₁₋₃ alkyl) -C₇₋₁₄ aralkyl group, each of which may be optionally substituted; the compound in which R^{1c} is a (carboxy-C₁₋₄ alkyl)-C₆₋₁₀ aryl group which maybe optionally substituted;
the compound in which R^{1c} is a (carboxy-C₂₋₃ alkyl) -C₆₋₁₀ aryl group which may be optionally substituted;
the compound in which R^{1c} is a (carboxy-C₂₋₃ alkyl)-phenyl group which may be optionally substituted ;
the compound in which R^{1c} is a (carboxyfuryl)-alkyl group which may be optionally substituted;
the compound in which R^{2c} is a C₃₋₆ alkyl group which have alkanoyloxy group and/or a hydroxy group;
the compound in which R^{2c} is a C₃₋₆ alkyl group which may have 1 to 3 substituents selected from a hydroxy group, acetoxy, propionyloxy, tert-butoxycarbonyloxy or palmitoyloxy;
the compound in which R^{2c} is 2,2-dimethylpropyl, 3-hydroxy-2,2-dimethylpropyl or 3-acetoxy-2,2-dimethylpropyl;
the compound in which R^{3c} is a methyl group;
the compound in which W is a chlorine atom;
the compound having R-configuration at 3-position, and S-configuration at 5-position.

In the aforementioned the formula, R^{1c} represents an optionally substituted 1-carboxyethyl group, an optionally substituted carboxy-C₃₋₆ linear alkyl group, an optionally substituted C₃₋₆ linear alkyl-sulfonyl group, an optionally substituted (carboxy-C₅₋₇ cycloalkyl)-C₁₋₃ alkyl group, or formula -X^{1c}-X^{2c}-Ar-X^{3c}-X^{4c}-COOH (wherein X^{1c} and X^{4c} respectively represents a bond or a C₁₋₄ alkylene group which may have substituents, X^{2c} and X^{3c} respectively represents a bond, -O- or -S- and Ar is an optionally substituted divalent aromatic heterocyclic group. However, when X^{1c} is a bond, X^{2c} represents a bond, and when X^{4c} is a bond, X^{3c} represents a bond).

Examples of the C₃₋₆ linear alkyl group in the optionally substituted carboxy-C₃₋₆ linear alkyl group represented by R^{1c} include n-propyl, n-butyl, n-pentyl, n-hexyl. Among these, n-propyl and n-butyl are preferable, and n-propyl is more preferable.

Examples of the C₃₋₆ linear alkyl group in the optionally substituted C₃₋₆ linear alkyl-sulfonyl group represented by R^{1c} include n-propyl, n-butyl, n-pentyl, n-hexyl. Among these, n-propyl and n-butyl are preferable, and n-propyl is more preferable.

Examples of the C₅₋₇ cycloalkyl group in the optionally substituted (carboxy-C₅₋₇ cycloalkyl)-C₁₋₃ alkyl group represented by R^{1c} include cyclopentyl, cyclohexyl and cycloheptyl. Among these, cyclopentyl and cyclohexyl are preferable, and cyclohexyl is more preferable.

Examples of the C₁₋₃ alkyl group in the optionally substituted (carboxy-C₅₋₇ cycloalkyl)-C₁₋₃ alkyl group represented by R^{1c} include methyl, ethyl, n-propyl and isopropyl. Among these, methyl and ethyl are preferable, and methyl is more preferable.

In the group represented by formula -X^{1c}-X^{2c}-Ar-X^{3c}-X^{4c}-COOH in R^{1c}, examples of the "C₁₋₄ alkylene group" in the "optionally substituted C₁₋₄ alkylene group" represented by X^{1c} and X^{4c} include methylene, dimethylene, trimethylene, tetramethylene, and preferably C₁₋₃ alkylene group, and among them the linear one may be preferably used.

Examples of the "divalent aromatic ring group" in the "optionally substituted divalent aromatic ring group" represented by Ar include a divalent aromatic hydrocarbon group or a divalent aromatic heterocyclic group.

Herein, examples of the divalent aromatic hydrocarbon group include a group formed by removing one hydrogen atom from C₆₋₁₀ aryl group (e.g., phenyl, naphthyl, etc.), and phenylene is preferably used as a divalent aromatic hydrocarbon group.

Examples of divalent aromatic heterocyclic group include a group formed by removing one hydrogen atom from the aromatic heterocyclic group, which contains at least 1 (preferably 1 to 4, more preferably 1 to 2) of 1 to 3 (preferably 1 to 2) hetero atoms selected from an oxygen atom, a sulfur atom and a nitrogen atom as an atom constituting a ring-system (annular atom).

Herein, examples of the aromatic heterocyclic group include a 5- to 6-membered aromatic monocyclic heterocyclic group such as furyl, thienyl, pyrrolyl, oxazolyl, isoxazolyl, thiazolyl, isothiazolyl, imidazolyl, pyrazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pyridazinyl, pyrimidinyl, pyrazinyl, triazinyl (preferably, furyl, thienyl, pyrrolyl, imidazolyl, thiazolyl, pyridyl, etc.) and an 8- to 12-membered aromatic fused heterocyclic group such as benzofuranyl, isobenzofuranyl, benzo[b]thienyl, indolyl, isoindolyl, 1H-indazolyl, benzimidazolyl, benzoxazolyl, 1,2-benzoisoxazolyl, benzothiazolyl, benzopyranyl, 1,2-benzoisothiazolyl, 1H-benzotriazolyl, quinolyl, isoquinolyl, cinnolyl, quinazolinyl, quinoxalinyl, phthalazinyl, naphthyridinyl, purinyl, pteridinyl, carbazolyl, α-carbolinyl, β-carbolinyl, γ-carbolinyl, acridinyl, phenoxazinyl, phenothiazinyl, phenazinyl, phenoxathiinyl, thianthrenyl, phenathridinyl, phenanthrolinyl, indolizinyl, pyrrolo[1,2-b]pyridazinyl, pyrazolo[1,5-a]pyridyl, imidazo [1,2-a]pyridyl, imidazo[1,5-a]pyridyl, imidazo[1,2-b]pyridazinyl, imidazo[1,2-a]pyrimidinyl, 1,2,4-triazolo[4,3-a]pyridyl, 1,2,4-triazolo[4,3-b]pyridazinyl (preferably, a heterocyclic group in which the aforementioned 5- to 6-membered aromatic monocyclic heterocyclic group is fused with a benzene ring, or a heterocyclic group in which two heterocyclic groups, the same or different, selected from the aforementioned 5- to 6-membered aromatic monocyclic heterocyclic group are fused, and more preferably, a heterocyclic group in which the aforementioned 5- to 6-aromatic monocyclic heterocyclic group is fused with a benzene ring).

The substituent of "C₁₋₄ alkylene group" in the "optionally substituted C₁₋₄ alkylene group" represented by X^{1c} and X^{4c}; and the substituent of "divalent aromatic ring group" in the "optionally substituted divalent aromatic ring group" include: (i) carboxyl group optionally esterified with a C₁₋₆ alkyl group or a C₆₋₁₀ aryl-C₁₋₄ alkyl group (e.g., methyl, ethyl, propyl, isopropyl, butyl, tert-butyl, phenyl, benzyl, etc.); (ii) phosphate group optionally mono- or di-substituted with C₁₋₆ alkyl (e.g., methyl, ethyl, n-propyl, isopropyl, n-butyl, isobutyl, n-pentyl, isopentyl, neopentyl, hexyl, etc.) or C₂₋₇ alkanoyloxy-C₁₋₆ alkyl such as acetoxymethyl and pivaloyloxymethyl group; (iii) a sulfonate group; (iv) sulfonamide group optionally substituted with a C₁₋₆ alkyl group or a C₆₋₁₀ aryl-C₁₋₄ alkyl group (e.g., methyl, ethyl, propyl, isopropyl, butyl, tert-butyl, benzyl, etc.); (v) hydroxy group and a sulfhydryl group, each optionally alkylated with a C₁₋₃ alkyl group (e.g., methyl, ethyl, propyl, etc.); (vi) a carbamoyl group; (vii) phenyl group optionally substituted with 1 to 5 substituents [e.g. a hydroxy group, chlorine, fluorine, an aminosulfonyl group, and amino group optionally substituted with a C₁₋₃ alkyl group (e.g. methyl, ethyl, propyl, etc.)], and may be attached to through O or S; (viii) amino group optionally mono- or di-substituted with a C₁₋₃ alkyl group (e.g. methyl, ethyl, propyl, etc.); (ix) cyclic amino group optionally substituted with 1 to 3 C₁₋₃ alkyl group (e.g., methyl, ethyl, etc.), benzyl, phenyl, and the like (e.g. a 5- to 6-membered cyclic amino group which may contain an oxygen atom or a sulfur atom in addition to a nitrogen atom as an annular atom of the cyclic amino group derived from a cyclic amine (by removing a hydrogen atom) such as piperidine, pyrrolidine, morpholine, thiomorpholine, piperazine, 4-methylpiperazine, 4-benzylpiperazine, 4-phenylpiperazine, 1,2,3,4-tetrahydroisoquinoline, phthalimide, etc.); (x) a 5- to 6-membered aromatic heterocyclic group (e.g., pyridyl, imidazolyl, indolyl, tetrazolyl, etc.) which may contain 1 to 4 hetero atoms selected from N, O or S, and may be attached to through O or S; (xi) a halogen atom (e.g., chlorine, fluorine, bromine, iodine, etc.); (xii) an C₁₋₄ alkyl group (e.g., methyl, ethyl, propyl, isopropyl, butyl, tert-butyl, etc.), C₁₋₄ alkoxy group (e.g., methoxy, ethoxy, propoxy, isopropoxy, butoxy, t-butoxy, etc.) or C₁₋₄ alkylthio group (e.g., methylthio, ethylthio, propylthio, isopropylthio, butylthio, tert-butylthio, etc.), each optionally substituted with substituents selected from a C₁₋₄ alkoxy group, a C₁₋₄ alkylthio group, a carboxyl group or a phenyl group; (xiii) a C₅₋₇ cycloalkyl group (e.g., cyclopentyl, cyclohexyl, cycloheptyl, etc.); and (xiv) a C₁₋₇ alkanoyloxy (e.g., formyloxy, acetoxy, propionyloxy, butyryloxy, t-butoxycarbonyloxy, isobutyryloxy, valeryloxy, pivaloyloxy, etc.). These substituents may be substituted at 1 to 6, preferably 1 to 3 substitutable positions. In addition, two substituents may be bonded so as to form C₃₋₆ alkylene, C₃₋₆ alkyleneoxy, C₃₋₆ alkylenedioxy or the like, for example, when two adjacent substituents on a phenyl group are bonded to form C₄ alkylene, tetrahydronaphthalene group is formed.

Specific examples of the group represented by -X^{1c}-X^{2c}-Ar-X^{3c}-X^{4c}-COOH in R^{1c} include an optionally substituted (carboxy-heteroaryl)-C₁₋₄ alkyl group [preferably, an optionally substituted (carboxy-furyl)-C₁₋₄ alkyl group], an optionally substituted (carboxy-C₆₋₁₀ aryl-C₁₋₄ alkyl group), an optionally substituted carboxy-heteroaryl group, an optionally substituted carboxy-C₆₋₁₀ aryl group, an optionally substituted (carboxy-C₁₋₄ alkyl)-heteroaryl group, an optionally substituted (carboxy-C₁₋₄ alkyl)-C₆₋₁₀ aryl group [preferably, a (carboxy-C₂₋₃ alkyl)-C₆₋₁₀ aryl group], an optionally substituted (carboxy-C₁₋₄ alkyl)-heteroaryl-C₁₋₄ alkyl group, an optionally substituted (carboxy-C₁₋₄ alkyl)-C₇₋₁₄ aralkyl group [preferably, a (carboxy-C₁₋₃ alkyl)-C₇₋₁₄ aralkyl group], an optionally substituted (carboxy-C₁₋₄ alkoxy)-C₆₋₁₀ aryl group, an optionally substituted (carboxy-C₁₋₄ alkoxy)-C₆₋₁₀ aryl-C₁₋₄ alkyl group, an optionally substituted (carboxy-C₁₋₄ alkyl)-C₆₋₁₀ aryloxy-C₁₋₄ alkyl group, an optionally substituted (carboxy-C₆₋₁₀ aryloxy)-C₁₋₄ alkyl group and an optionally substituted (carboxy-C₁₋₄ alkylthio)-heteroaryl group.

Herein, the same group as the aforementioned "aromatic heterocyclic group" may be exemplified for heteroaryl, and the heteroaryl may have the same substituent as the substituent which the aforementioned "aromatic heterocyclic group" may have. In addition, examples of C₆₋₁₀ aryl include phenyl, naphthyl, azulenyl, with phenyl being preferably used. The C₆₋₁₀ aryl may have the same substituent as the substituent which the aforementioned "aromatic heterocyclic group" may have. Examples of the alkyl group in the optionally substituted (carboxyfuryl)-C₁₋₄ alkyl group represented by R¹ include, for example, C₁₋₄ linear or branched alkyl group such as methyl, ethyl, n-propyl, isopropyl, n-butyl, isobutyl, 1,1-dimethylethyl, and the like. Among these, a C₁₋₄ alkyl group such as methyl, ethyl, n-propyl, isopropyl and n-butyl are preferable, and methyl, ethyl, n-propyl are more preferable. Examples of the carboxyfuryl group include, for example, 3-carboxy-2-furyl, 4-carboxy-2-furyl, 2-carboxy-3-furyl, 2-carboxy-5-furyl. Among these, 3-carboxy-2-furyl and 4-carboxy-2-furyl are preferable, and 3-carboxy-2-furyl is more preferable.

Examples of the C₂₋₃ alkyl group in the optionally substituted (carboxy-C₂₋₃ alkyl)-C₆₋₁₀ aryl group represented by R^{1c} include ethyl, n-propyl, isopropyl, with ethyl and n-propyl being preferable. Examples of the C₆₋₁₀ aryl group include phenyl, naphthyl, azulenyl, with phenyl being preferable.

Examples of the C₁₋₃ alkyl group in the optionally substituted (carboxy-C₁₋₃ alkyl) -C₇₋₁₄ aralkyl group represented by R^{1c} include methyl, ethyl, n-propyl and isopropyl, with methyl and ethyl being preferable and ethyl being particularly preferable. Examples of a C₇₋₁₄ aralkyl group (a C₆₋₁₀ aryl-C₁₋₄ alkyl group) include phenylmethyl, 1-phenylethyl, 2-phenylethyl, 3-phenylpropyl, 2-phenylpropyl, 4-phenylbutyl, (1-naphthyl)methyl, (2-naphthyl)methyl, 1-(1-naphthyl)ethyl, 1-(2-naphthyl)ethyl, 3-(1-naphthyl)propyl, 3-(1-naphthyl)propyl, 4-(1-naphthyl)butyl, 4-(2-naphthyl)butyl, with phenylmethyl, 1-phenylethyl, 3-phenylpropyl, (1-naphthyl)methyl, (2-naphthyl)methyl, (1-naphthyl)ethyl and (2-naphthyl)ethyl being preferable, and phenylmethyl and 2-phenylethyl being particularly preferable.

As for the substituent, in the case each group represented by R^{1c} have a substituent, the same substituent as that "divalent aromatic heterocyclic group", in the "optionally substituted divalent aromatic heterocyclic group" represented by Ar, may have is exemplified, , and these substituents can exist on 1 to 6, preferably 1 to 3 substitutable positions. For each group represented by R^{1c} it is preferable that the carboxylic portion is unsubstituted, and an arbitrary portion other than the carboxylic portion may have a substitutable substituent at a substitutable position.

As for R^{1c}, a 3-carboxypropyl group, a 1-carboxyethyl group, or a C₃₋₆ linear alkyl-sulfonyl group , a (carboxy-C₅₋₇ cycloalkyl)-C₁₋₃ alkyl group, a (carboxyfuryl) -alkyl group, a carboxy-C₆₋₁₀ aryl group, a (carboxy-C₁₋₄ alkyl)-C₆₋₁₀ aryl group [preferably, a (carboxy-C₂₋₃ alkyl)-C₆₋₁₀ aryl group], or a (carboxy-C₁₋₃ alkyl)-C₇₋₁₄ aralkyl group, each of which may have substituent and the like are preferable, an optionally substituted (carboxy-C₁₋₄ alkyl)-C₆₋₁₀ aryl group is preferable, and an optionally substituted (carboxy-C₂₋₃ alkyl)-C₆₋₁₀ aryl group is more preferable. Among these, an optionally substituted (carboxy-C₂₋₃ alkyl) phenyl group is preferable.

Examples of the C₃₋₆ alkyl group in the C₃₋₆ alkyl group optionally substituted with an alkanoyloxy group or a hydroxy group represented by R^{2c} , include n-propyl, isopropyl, 1,1-dimethylethyl, n-butyl, isobutyl, n-pentyl, 2,2-dimethylpropyl, isopentyl, n-hexyl, isohexyl. Among these, isopropyl, 1,1-dimethylethyl, n-butyl, isobutyl, 2,2-dimethylpropyl, isohexyl are preferable, and 2,2-dimethylpropyl is particularly preferable.

Examples of the alkanoyloxy group in the C₃₋₆ alkyl group optionally substituted with an alkanoyloxy group or a hydroxy group represented by R^{2c} include a C₁₋₂₀ alkanoyloxy group such as formyloxy, acetoxy, propionyloxy, butyryloxy, tert-butoxycarbonyloxy, isobutyryloxy, valeryloxy, pivaloyloxy, lauryloxy, palmitoyloxy, stearoyloxy (preferably a C₁₋₇ alkanoyloxy group, etc.). Among these, acetoxy, propionyloxy, tert-butoxycarbonyloxy, palmitoyloxy are preferable, and acetoxy is particularly preferable. 1 to 3 of the alkanoyloxy groups or the hydroxy groups may be substituted at a substitutable position.

Preferable examples of C₃₋₆ alkyl group optionally substituted with an alkanoyloxy group or a hydroxy group represented by R^{2c} include, for example, 2,2-dimethylpropyl, 3-hydroxy-2,2-dimethylpropyl, 3-hydroxy-2-hydroxymethyl-2-methylpropyl, 3-acetoxy-2,2-dimethylpropyl, 3-acetoxy-2-hydroxymethyl-2-methylpropyl and 3-acetoxy-2-acetoxymethyl-2-methylpropyl. Among these, 2,2-dimethylpropyl, 3-hydroxy-2,2-dimethylpropyl, 3-acetoxy-2,2-dimethylpropyl is particularly preferable.

In addition, as R^{2c} a C₃₋₆ alkyl group having an alkanoyloxy group and/or a hydroxy group is preferable.

Examples of the lower alkyl group represented by R^{3c} include a C₁₋₆ alkyl group such as methyl, ethyl, n-propyl, isopropyl, n-butyl, tert-butyl, pentyl, hexyl. Among these, a C₁₋₃ alkyl group is preferable. In view of the pharmacological activity, a methyl group is particularly preferable as R^{3c}.

Examples of the halogen atom represented by W include chlorine, fluorine, bromine and iodine atoms. Among these, a chlorine atom is preferable.

The present invention includes the compound represented by the formula (Ic) in the form of either free or a pharmacologically acceptable salt thereof. As such salt, when the compound represented by the formula (Ic) has an acidic group such as a carboxyl group or the like, it may form a salt with an inorganic base (e.g., alkali metals such as sodium and potassium, alkaline earth metals such as calcium and magnesium, transition metals such as zinc, iron and copper, etc.) or an organic base (e.g., organic amines such as trimethylamine, triethylamine, pyridine, picoline, ethanolamine, diethanolamine, triethanolamine, dicyclohexylamine, N,N'-dibenzylethylenediamine, and basic amino acids such as an arginine, lysine, ornithine, etc.).

In the case where the compound represented by the formula (Ic) of the present invention has a basic group such as an amino group or the like, it may form a salt with inorganic or organic acid (e.g., hydrochloric acid, nitric acid, sulfuric acid, phosphoric acid, carbonic acid, bicarbonic acid, formic acid, acetic acid, propionic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid, etc.), acidic amino acid such as aspartic acid, glutamic acid, and the like.

The compound represented by the formula (Ic) or salts thereof has asymmetric carbon atoms at 3- and 5-position, but they may be in a mixture of the steroisomers, and the isomers may also be separated by conventional means. The trans isomer wherein the substituents on 3- and 5-positions exist in the opposite direction relative to the surface of a 7-membered ring, is preferable, and the isomer wherein the absolute configuration at 3-position being R-configuration, and the absolute configuration at 5-position being S-configuration is particularly preferable. They may also be in a racemic or optically active form. The optically active form can be separated from the racemic form by the conventional optical resolution means.

As the compounds represented by the formula (Ic) and salts thereof of the present invention, the following compounds are preferably exemplified.

N-propanesulfonyl-[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetamide or salts thereof; (2R)-2-[[(3R,5S)-7-chloro-5-(2,3-dimethoxyphenyl)-1-(2,2-dimethylpropyl)-2-oxo-1,2,3,5-tetrahydro-4,1-benzoxazepin-3-yl]acetyl]aminopropionic acid or salts thereof; 3-[3-[[(3R,5S)-7-chloro-5-(2,3-dimethoxyphenyl)-1-(2,2-dimethylpropyl)-2-oxo-1,2,3,5-tetrahydro-4,1-benzoxazepin-3-yl]acetyl]aminophenyl]propionic acid or salts thereof; 4-[[(3R,5S)-7-chloro-5-(2,3-dimethoxyphenyl)-1-(2,2-dimethylpropyl)-2-oxo-1,2,3,5-tetrahydro-4,1-benzoxazepin-3-yl]acetyl]aminobutanoic acid or salts thereof; trans-4-[[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetyl]-aminomethyl-1-cyclohexane carboxylic acid or salts thereof; trans-4-[[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl]-aminomethyl-1-cyclohexanecarboxylic acid or salts thereof; 3-[3-[[[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetyl]amino]-4-fluorophenyl]propionic acid or salts thereof; 3-[3-[[[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl]amino]-4-methylphenyl]propionic acid or salts thereof; 3-[3-[[[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetyl]amino]-4-methylphenyl]propionic acid or salts thereof; 3-[3-[[[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl]aminomethyl]phenyl]propionic acid or salts thereof; 3-[3-[[[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetyl]aminomethyl]phenyl]propionic acid or salts thereof; 3-[3-[[[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl]amino]-4-methoxyphenyl]propionic acid or salts thereof; 2-[2-[[[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl]amino]ethyl]furan-3-carboxylic acid or salts thereof; 3-[3-[[[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl]amino]-4-fluorophenyl]propionic acid or salts thereof; 3-[3-[[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl]aminophenyl]propionic acid or salts thereof; 4-[3-[[[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl]amino]-4-methoxyphenyl]butanoic acid or salts thereof; 5-[3-[[[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl]amino]-4-methoxyphenyl]pentanoic acid or salts thereof; 5-[3-[[[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl]amino]-4-fluorophenyl]pentanoic acid or salts thereof.

The compound represented by the above-mentioned formula (Ic) or a salt thereof can be produced, for example, according to a method disclosed in EP A 567,026, WO95/21834 (international application based on Japanese Patent Application No. 6-15531), EP A 645,377 (application based on Japanese Patent Application No. 6-229159), EP A 645,378 (application based on Japanese Patent Application No. 6-229160), WO01/98282 (international application based on Japanese Patent Application No. 2000-190253) and the like, or an equivalent method.

As the starting compound of the compound represented by the formula (I) of the present invention, the salts as mentioned above can be used, but they are not particularly limited as long as they do not interfere with the reaction.

The "skeletal muscle protection" in the present invention refers to therapeutic or preventive effects for the symptoms that the skeletal muscle is in necrosis or myolysis caused by various factors, for example, ischemia, labor, excessive exercise, trauma (bruise, skeletal muscle bleeding, electric shock), burn, malignant hyperthermia, malignant syndrome, metabolic myopathy, inflammatory myopathy, muscular dystrophy, infection, poisoning, abnormal metabolism, hyperthermia and the like. More specifically, it refers to an action of protecting skeletal muscle from cell disorder caused by these factors (skeletal muscle protecting action based on suppression of the cell disorder caused by these factors). Furthermore, it also comprises therapeutic or preventive effects for myalgia by cytotoxicity of other medicines (for example, an HMG-CoA reductase inhibitor, cyclosporin, fibrate-related medicine and the like), and rhabdomyolysis as further serious case.

Particularly, the agent of the present invention shows excellent therapeutic or preventive effects for myalgia, and further serious rhabdomyolysis which are developed by an HMG-CoA reductase inhibitor, and suppresses the decrease of a geranylgeranylated metabolite (for example, geranylgeranyl pyrophosphate, geranylgeraniol, geranylgeranylated protein and the like) in a muscular cell, which is caused by the HMG-CoA reductase inhibitor. Herein, the HMG-CoA reductase inhibitor includes atorvastatin, lovastatin, simvastatin, pravastatin, rosuvastatin, itavastatin, fluvastatin, cerivastatin, and pitavastatin

The agent of the present invention has an excellent skeletal muscle protecting action, and further is low toxic and safe (for example, more excellent as a drug in view of acute toxicity, chronic toxicity, genetic toxicity, cardiac toxicity, drug interaction, carcinogenicity and the like). Accordingly, the agent of the present invention can be used safely as, for example, a preventive and/or therapeutic agent for rhabdomyolysis, a preventive and/or therapeutic agent for myoglobinuria accompanied with rhabdomyolysis, a preventive and/or therapeutic agent for myalgia and the like in a mammal (for example, mouse, rat, hamster, rabbit, cat, dog, cattle, horse, sheep, monkey, human and the like).

In the agent of the present invention, the compound having inhibitory activity against squalene synthase or a salt thereof, or a prodrug thereof (hereinafter, also referred to as an "SSI compound or a prodrug thereof") which is an active ingredient, can be administered as bulk powder, or usually in the form of a pharmaceutical composition or preparation which is prepared by a conventional method using conventional carriers for formulation in suitable amount, which carriers are suitably selected from, for example, an excipient (for example, calcium carbonate, kaolin, sodium hydrogen carbonate, lactose, starches, crystalline cellulose, talc, granulated sugar, porous substances, etc.), a binder (for example, dextrin, gums, alcoholized starch, gelatin, hydroxypropylcellulose, hydroxypropylmethylcellulose, pullulan, etc.), a disintegrating agent (for example, carboxymethylcellulose calcium, sodium croscarmellose, crospovidone, low-substituted hydroxypropylcellulose, partially pregelatinated starch, etc.), a lubricant (for example, magnesium stearate, calcium stearate, talc, starch, sodium benzoate, etc.), a colorant (for example, tar dye, caramel, iron sesquioxide, titanium oxide, riboflavins, etc.), a flavoring substance (for example, sweeters, flavors, etc.), a stabilizer (for example, sodium sulphite, etc.), a preservative (for example, parabens, sorbic acid, etc.) and the like. The agent of the present invention including the above-mentioned preparations contains suitably the SSI compound or prodrug thereof in an effective amount for treating and preventing the diseases. The content of the SSI compound or prodrug thereof in the preparation of the present invention, is usually 0.1 to 100% by weight based on the total preparation. Furthermore, the preparation used in the present invention may contain other drug ingredients as active ingredients, in addition to the SSI compound or prodrug thereof. Such ingredient is not particularly limited as long as the object of the present invention is achieved, and can be used in a suitable mixing ratio. Specific examples of the preparations include tablets (including sugar-coated tablets and film-coated tablets), pills, capsules, granules, fine-granules, powders, syrup, emulsion, suspension, injectable preparation, sustained release injectable preparation, inhalant, ointment, etc. These preparations are prepared by a conventional method (for example, a method described in Japanese Pharmacopoeia).

Specifically, tablets can be prepared by granulating suitably the SSI compound or a prodrug thereof as it is, or with an excipient, a binder, a disintegrating agent, or other suitable additives, which are added and homogenously kneaded, and then compressing and molding it with adding a lubricant and the like. Alternatively, tablets can be prepared by directly compressing and molding the SSI compound or a prodrug thereof as it is, or with an excipient, a binder, a disintegrating agent, or other suitable additives, which are added and homogenously kneaded, or by compressing and molding granules previously prepared as they are, or with suitable additives, which are added and homogenously kneaded. Furthermore, the present preparation can contain a colorant, a flavoring substance and the like, if necessary. Furthermore, the present preparation can be coated with a suitable coating agent. The injectable preparation can be prepared by dissolving, suspending or emulsifying a certain amount of the SSI compound or a prodrug thereof in an aqueous solvent such as an injectable solvent, a physiological saline solution, a Ringer's solution and the like, or in a non-aqueous solvent such as a vegetable oil usually, to prepare a certain amount of the injectable solution, or, by sealing a certain amount of the SSI compound or a prodrug thereof in a vessel for injection.

Carriers for oral preparations include substances conventionally used in the field of a formulation such as starch, mannitol, crystalline cellulose and carboxymethylcellulose sodium. Carriers for injection include, for example, distilled water, a physiological saline solution, a glucose solution, infusion solution and the like. Other additives which are used for general formulations can be suitably added.

Furthermore, the preparation of the present invention can be used as a sustained-release preparation. The sustained-release preparation can be administered as microcapsules (for example, microsphere/microcapsules, micro-particles and the like) as they are, which are prepared by a method such as drying-in-water method (o/w method, w/o/w method and the like), phase separation method, spray drying method or a similar method thereto, or as other various preparations formulated starting from a pharmaceutical composition in the form of microcapsules, or spheres, needles, pellets, film or cream. The dosage form includes parenteral preparations (for example, an injectable preparation or an implant for intramuscular, subcutaneous, organ; an intramucosal preparation for nasal cavity, rectum, uterus and the like), oral preparations (for example, hard capsules, soft capsules, granules, powders, suspension and the like) and the like.

When the sustained-release preparation is an injectable preparation, it is prepared as an aqueous suspension prepared by dispersing microcapsules with a dispersant (for example, a surfactant such as Tween 80 and HCO-60; polysaccharide such as carboxymethylcellulose, sodium alginate and sodium hyaluronate; protamine sulfate, polyethylene glycol and the like), a preservative (for example, methylparaben, propylparaben and the like), an isotonic agent (for example, sodium chloride, mannitol, sorbitol, glucose and the like), a local anesthetic (for example, xylocaine hydrochloride, chlorobutanol and the like), or as an oily suspension prepared by dispersing them in a vegetable oil (for example, sesame oil, corn oil and the like) or in a mixture thereof with phospholipid (for example, lecithin and the like), or with middle chain triglyceride (for example, Miglyol 812 and the like).

When the sustained-release preparation is microcapsules, the mean particle diameter is about 0.1 to about 300 µm, preferably, about 1 to about 150 µm, more preferably about 2 to about 100 µm.

A method of preparing the microcapsules aseptic preparations includes a method wherein all the processes are conducted under aseptic conditions, sterilization with gamma rays, the addition of an antiseptic and the like, which are not particularly limited thereto.

Dose of the agent of the present invention is varied depending on an administration route, symptoms, the age or body weight of the patient and the like. For example, when orally administered to an adult patient as a skeletal muscle protecting agent, it is preferable to administer 1 to 400 mg/day, preferably 6 to 120 mg/day as the SSI compound once or several times a day. The administration route may be oral or parenteral.

Furthermore, the dose of the sustained-release preparation as an example of the agent of the present invention is varied depending on the duration of release as well as the administration route, symptoms, the age or weight of the patient and the like. However, it is not particularly limited if it is an amount to maintain the effective concentration of the active ingredient in the body and the number of administration can be suitably selected depending on the situation, for example, once a day to once 3 days, or once a week to once 3 months.

When the skeletal muscle protecting agent of the present invention is used for protecting skeletal muscle from cytotoxicity of an HMG-CoA reductase inhibitor, the administration mode of the SSI compound and the HMG-CoA reductase inhibitor used in the present invention is not particularly limited, and the SSI compound and the HMG-CoA reductase inhibitor may be combined at the time of administration. Examples of such administration mode include the following methods:
(1) administration of a single preparation prepared by formulating the SSI compound and the HMG-CoA reductase inhibitor simultaneously (2) simultaneous administration of two kinds of preparations obtained by formulating the SSI compound and the HMG-CoA reductase inhibitor separately, via a single administration route, (3) separate administration at an interval of two kinds of preparations obtained by formulating the SSI compound and the HMG-CoA reductase inhibitor separately, via a single administration route, (4) simultaneous administration of two kinds of preparations obtained by formulating the SSI compound and the HMG-CoA reductase inhibitor separately, via different administration routes, (5) separate administration at an interval of two kinds of preparations obtained by formulating the SSI compound and the HMG-CoA reductase inhibitor separately, via different administration routes (e.g. administration of the SSI compound followed by the HMG-CoA reductase, or administration in the reverse order). Dose of an HMG-CoA reductase inhibitor can be appropriately selected based on the dose which is clinically used. The compounding ratio of the SSI compound and an HMG-CoA reductase inhibitor can be appropriately selected depending on the administration subject, administration route, target diseases, symptoms, combinations thereof, etc. For example, if the administration subject is human, the SSI compound may be used in an amount of 0.01 to 100 parts by weight to 1 part by weight of the HMG-CoA reductase inhibitor.

The agent of the present invention has an excellent skeletal muscle protecting action, and for example, it has excellent preventive and/or therapeutic effects for myalgia or rhabdomyolysis which is developed by other medicines such as the HMG-CoA reductase inhibitor.

Hereinafter, test results showing the pharmacological effects of the agent of the present invention are described.

### Test compound 1:

### N-[[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetyl]piperidine-4-acetic acid

Test compound 1 is a compound described in Example 36 of JP-A No. 2002-080468, and can be synthesized by the method described in this publication and the like.

### Test compound 2:

### 3-[3-[[[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl]amino]phenyl]propionic acid

Test compound 2 is a compound described in Example 36 of JP-A No. 9-136880, and can be synthesized by the method described in this publication and the like. Furthermore, it has been described in Test 1 of the publication that the test compound 2 has inhibitory activity against squalene synthase.

### Test compound 3:

### N-[[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl]piperidine-4-acetic acid

Test compound 3 is a compound described as compound No. 13-1 in JP-A No. 9-136880, and can be synthesized by the method described in this publication and the like. Furthermore, it has been described in Test 1 of the publication that the test compound 3 has inhibitory activity against squalene synthase.

### Test Example 1

### Increasing effect on geranylgeraniol (GGOH) content in soleus muscle

### Method:

To 6 week-old male SD rat (4 per group) was orally administered forcedly by means of a stomach sonde a vehicle or Test compounds 1 or 2 at a dose of 10 mL/kg once a day for 14 days so as to be in an amount of 40 or 200 mg/kg. In the next morning of the 14th administration, the rat was killed under ether anesthesia. Immediately, the soleus muscle was collected and frozen on a dry ice, and then kept at -80°C. Later, to the muscle homogenate was added phosphatase for dephosphorylation, and then the concentration of geranylgeraniol (sum total of geranylgeranyl pyrophosphate and geranylgeraniol) in the muscle was determined with LC/MS/MS (Table 1).

### Results:

**Table 1**

| Treatment | Dose (mg/kg) | GGOH content in the soleus muscle (µg/g soleus muscle) |
|---|---|---|
| Vehicle | 0 | 0.190 ± 0.007 |
| Test compound 1 | 40 | 0.224 ± 0.017 |
| Test compound 1 | 200 | 0.255 ± 0.027* |
| Test compound 2 | 40 | 0.225 ± 0.019 |
| Test compound 2 | 200 | 0.255 ± 0.006* |
| Data represent Mean ± SE (N=4). | | |

| | | |
|---|---|---|
| *P < 0.025 vs. Control (one-tailed Williams' test) | | |

Results of Table 1 showed that the SSI compound increased the geranylgeraniol content in the skeletal muscle.

### Test Example 2

### Effect of geranylgeranyl pyrophosphate (GGPP) on cytotoxicity of HMG-CoA reductase inhibitor

### Method:

Human normal skeletal muscle cells (Bio Whittaker) having passage number 6 were cultured on a 96-well plate in a SkGM medium (Bio Whittaker), and were divided into Groups 1 to 6 shown in Table 1 in a confluent state. The drugs in Treatments 1 and 2 were added at the same time, and after 3 days, the content of ATP in the cell was measured with an ATP Lite-M kit (Packard), and the number of live cells was measured (Table 2).

### Results:

**Table 2**

| | Treatment 1 | Treatment 2- | The number of the live cells (% of Group 1) |
|---|---|---|---|
| Group 1 | Vehicle | Vehicle | 100 ± 1.3 |
| Group 2 | Vehicle | GGPP (10 µM) | 93.5 ± 0.8 |
| Group 3 | Simvastatin (10 µM) | Vehicle | 46.2 ± 1.5# |
| Group 4 | Simvastatin (10 µM) | GGPP (10 µM) | 72.3 ± 1.6* |
| Group 5 | Atorvastatin (10 µM) | Vehicle | 52.0 ± 0.9# |
| Group 6 | Atorvastatin (10 µM) | GGPP (10 µM) | 67.8 ± 1.3$ |
| Data represent Mean ± SE (N=3). | | | |

| | | | |
|---|---|---|---|
| #P < 0.025 vs Group 1 (one-tailed Williams' test) | | | |
| *P < 0.01 vs Group 3 (Student's t test) | | | |
| $P<0.01 vs Group 5 (Student's t test) | | | |

Results of Table 2 showed that geranylgeranyl pyrophosphate, which is an active form of geranylgeraniol in the living body that is increased by the SSI compound, had an activity reducing cytotoxicity in skeletal muscle cells.

### Test Example 3

### Effect of Test compound 3 on cytotoxicity of HMG-CoA reductase inhibitor (1)

### Method:

Human normal skeletal muscle cells (Bio Whittaker) having passage number 6 were cultured on a 96-well plate in a SkGM medium (Bio Whittaker), and were divided into Groups 1 to 4 shown in Table 3 as a confluent state. The drugs in Treatments 1 and 2 were added at the same time, and after 3 days, the content of ATP in the cell was measured with an ATP Lite-M kit (Packard), and the number of the live cells was measured (Table 3).

### Results:

**Table 3**

| | Treatment 1 | Treatment 2 | The number of the live cells (% of Group 1) |
|---|---|---|---|
| Group 1 | Vehicle | Vehicle | 100 ± 3.6 |
| Group 2 | Test compound 3 (10 µM) | Vehicle | 96 ± 1.2 |
| Group 3 | Vehicle | Atorvastatin (100 µM) | 34 ± 0.6# |
| Group 4 | Test compound 3 (10 µM) | Atorvastatin (100 µM) | 42 ± 0.5* |
| Data represent Mean ± SE (N=3). | | | |

| | | | |
|---|---|---|---|
| #P < 0.025 vs Group 1 (one-tailed Williams' test) | | | |
| *P < 0.05 vs Group 3 (Student's t test) | | | |

### Test Example 4

### Effect of Test compound 3 on cytotoxicity of HMG-CoA reductase inhibitor (2)

### Method:

Human normal skeletal muscle cells (Bio Whittaker) having passage number 6 were cultured on a 96-well plate in a SkGM medium (Bio Whittaker), and were divided into Groups 1 to 6 shown in Table 4 as a confluent state. The drug in Treatment 1 was added and after 1 day, the medium was completely removed. Then, the drug in Treatment 2 was added and after 3 days, the content of ATP in the cell was measured with an ATP Lite-M kit (Packard), and the number of the live cells was measured (Table 4).

### Results:

**Table 4**

| | Treatment 1 | Treatment 2 | The number of the live cells (% of Group 1) |
|---|---|---|---|
| Group 1 | Vehicle | Vehicle | 100 ± 0.5 |
| Group 2 | Test compound 3 (10 µM) | Vehicle | 98 ± 2.3 |
| Group 3 | Vehicle | Atorvastatin (10 µM) | 57 ± 1.1# |
| Group 4 | Test compound 3 (10 µM) | Atorvastatin (10 µM) | 76 ± 1.8** |
| Group 5 | Vehicle | Atorvastatin (100 µM) | 35 ± 1.9# |
| Group 6 | Test compound 3 (10 µM) | Atorvastatin (100 µM) | 45 ± 0.2* |
| Data represent Mean ± SE (N=3). | | | |

| | | | |
|---|---|---|---|
| #P < 0.025 vs Group 1 (one-tailed Williams' test) | | | |
| **P < 0.01 vs Group 3 (Student's t test) | | | |
| *P < 0.05 vs Group 5 (Student's t test) | | | |

The results of Tables 3 and 4 showed that the SSI compound had an excellent activity reducing cytotoxicity in atorvastatin-treated skeletal muscle cells.

### Preparation Example

The skeletal muscle protecting agent of the present invention can be produced, for example, by the following prescription.

In addition, for ingredients other than the active ingredients (additives) described in the following prescription, products described in Japanese Pharmacopoeia, Japanese Pharmaceutical Codex, or The Japanese Standards of Drug Additives can be used.

### 1. Capsule

| | | |
|---|---|---|
| (1) | N-[[(3R,5S)-7-chloro-5-(2,3-dimethoxyphenyl)-1-(3-hydroxy-2,2-dimethylpropyl)-2-oxo-1,2,3,5-tetrahydro-4,1- | |
| | benzoxazepin-3-yl]acetyl]piperidine-4-acetic acid | 10 mg |
| (2) | Lactose | 90 mg |
| (3) | Microcrystalline cellulose | 70 mg |
| (4) | Magnesium stearate | 10 mg |
| | 1 capsule | 180 mg |

(1), (2) and (3) and the half of (4) are kneaded and then granulated. To this is added the remaining (4), and the whole is sealed into a gelatin capsule.

### 2. Tablet

| | | |
|---|---|---|
| (1) | N-[[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl]piperidine-4-acetic acid | 10 mg |
| (2) | Lactose | 35 mg |
| (3) | Corn starch | 150 mg |
| (4) | Microcrystalline cellulose | 30 mg |
| (5) | Magnesium stearate | 5 mg |
| | 1 tablet | 230 mg |

(1), (2), (3), 2/3 of (4) and the half of (5) are kneaded and then granulated. The remaining (4) and (5) are added to the granules, and compressed and molded into tablets.

### 3. Injection

| | | |
|---|---|---|
| (1) | N-[[(3R,5S)-7-chloro-5-(2,3-dimethoxyphenyl)-1-(3- | |
| | hydroxy-2,2-dimethylpropyl)-2-oxo-1,2,3,5-tetrahydro-4,1-benzoxazepin-3-yl]acetyl]piperidine-4-acetic acid | 10 mg |
| (2) | Inositol | 100 mg |
| (3) | Benzyl alcohol | 20 mg |
| | 1 Ampoule | 130 mg |

(1), (2) and (3) are dissolved in distilled injection solvent so as to be a total of 2 ml, which is sealed into an ampoule. All steps are conducted under sterilized conditions.

### Industrial Applicability

The agent of the present invention has an excellent skeletal muscle protecting action, and for example, it has excellent preventive and/or therapeutic effects for myalgia or rhabdomyolysis which is developed by other medicines such as an HMG-CoA reductase inhibitor.

## Claims

1. A skeletal muscle protecting agent comprising a compound having inhibitory activity against squalene synthase or a salt thereof, or a prodrug thereof.

2. The agent according to claim 1, which is a skeletal muscle protecting agent which protects skeletal muscle from cell disorder.

3. The agent according to claim 1, which is a skeletal muscle protecting agent which protects skeletal muscle from cytotoxicity of other medicines.

4. The agent according to claim 3, wherein the other medicine is an HMG-CoA reductase inhibitor.

5. The agent according to claim 1, which is a preventive and/or therapeutic agent for myalgia or rhabdomyolysis.

6. The agent according to claim 1, wherein the compound having inhibitory activity against squalene synthase is a compound represented by the formula: 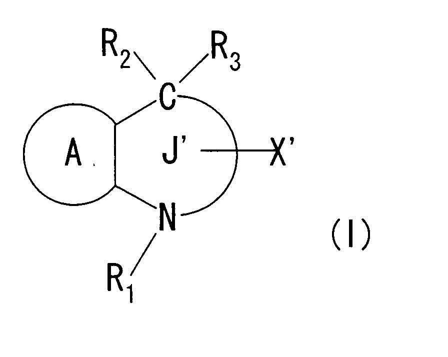 wherein R₁ is a hydrogen atom or an optionally substituted hydrocarbon group, R₂ and R₃ are the same or different and a hydrogen atom, an optionally substituted hydrocarbon group or an optionally substituted heterocyclic group, X' is a substituent comprising an optionally esterified carboxyl group, an optionally substituted carbamoyl group, an optionally substituted hydroxy group, an optionally substituted amino group or an optionally substituted heterocyclic residue having a hydrogen atom which can be deprotonated, Ring A is an optionally substituted benzene ring or an optionally substituted heterocyclic ring, Ring J' is a 7- or 8-membered heterocyclic ring having 3 or less hetero atoms, as atoms constituting a ring, and Ring J' may further have a substituent in addition to R₁, R₂, R₃ and X'.

7. The agent according to claim 1, wherein the compound having inhibitory activity against squalene synthase is a compound represented by the formula: 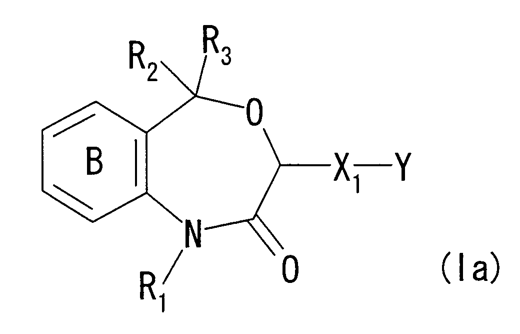 wherein R₁ is a hydrogen atom or an optionally substituted hydrocarbon group, R₂ and R₃ are the same or different and a hydrogen atom, an optionally substituted hydrocarbon group or an optionally substituted heterocyclic group, X₁ is a bond or divalent atomic chain, Y is an optionally esterified carboxyl group, an optionally substituted carbamoyl group, an optionally substituted hydroxy group, an optionally substituted amino group or an optionally substituted heterocyclic residue having a hydrogen atom which can be deprotonated, and Ring B is an optionally substituted benzene ring.

8. The agent according to claim 1, wherein the compound having inhibitory activity against squalene synthase is a compound represented by the formula: 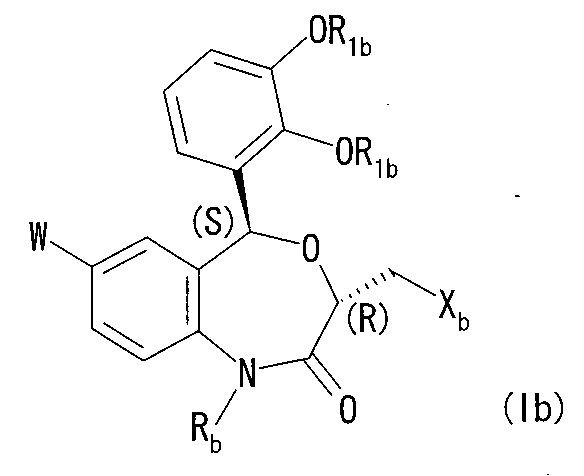 wherein R_{b} is a lower alkyl group optionally substituted with an optionally substituted hydroxy group, X_{b} is an optionally substituted carbamoyl group or an optionally substituted heterocyclic group having a hydrogen atom which can be deprotonated, R_{1b} is a lower alkyl group and W is a halogen atom.

9. The agent according to claim 8, wherein R_{b} is C₁₋₆ alkyl which may have 1 to 3 substituents selected from a hydroxy group, acetyloxy, propionyloxy, t-butoxycarbonyloxy, palmitoyloxy, dimethylaminoacetyloxy and 2-aminopropionyloxy.

10. The agent according to claim 8, wherein R_{1b} is methyl.

11. The agent according to claim 8, wherein W is a chlorine atom.

12. The agent according to claim 8, wherein X_{b} is a group represented by the formula: 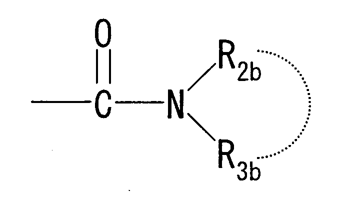 wherein R_{2b} and R_{3b} are each a hydrogen atom, an optionally substituted hydrocarbon group, an optionally substituted heterocyclic group or an acyl group, or R_{2b} and R_{3b} may form, together with the adjacent nitrogen atom, an optionally substituted 5- or 6-membered nitrogen-containing heterocyclic ring which may contain 1 to 3 hetero atoms selected from a nitrogen atom, a sulfur atom and an oxygen atom, as atoms constituting a ring.

13. The agent according to claim 8, wherein X_{b} is a group represented by the formula: 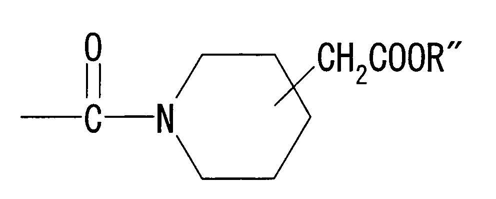 wherein R" is a hydrogen atom or C₁₋₄ alkyl.

14. The agent according to claim 1, wherein the compound having inhibitory activity against squalene synthase is N-[[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetyl]piperidine-4-acetic acid or N-[[(3R,5S)-7-chloro-5-(2,3-dimethoxyphenyl)-1-(3-hydroxy-2,2-dimethylpropyl)-2-oxo-1,2,3,5-tetrahydro-4,1-benzoxazepin-3-yl]acetyl]piperidine-4-acetic acid.

15. A skeletal muscle protecting agent comprising a compound having an action of suppressing the decrease of a geranylgeranylated metabolite in a muscular cell, or a salt thereof, or a prodrug thereof.

16. A method for protecting skeletal muscle, comprising administering an effective amount of a compound having inhibitory activity against squalene synthase, or a salt thereof, or a prodrug thereof to a mammal.

17. A method for protecting skeletal muscle, comprising administering an effective amount of a compound having an action of suppressing the decrease of a geranylgeranylated metabolite in a muscular cell, or a salt thereof, or a prodrug thereof to a mammal.

18. Use of a compound having inhibitory activity against squalene synthase, or a salt thereof, or a prodrug thereof for manufacturing a skeletal muscle protecting agent.

19. Use of a compound having an action of suppressing the decrease of a geranylgeranylated metabolite in a muscular cell, or a salt thereof, or a prodrug thereof for manufacturing a skeletal muscle protecting agent.
